# EUROPEAN PATENT SPECIFICATION

(11) **EP 2 761 019 B1**
(45) Date of publication and mention of the grant of the patent: **26.10.2016**
(21) Application number: 12762215.7
(22) Date of filing: 14.09.2012
(51) Int. Cl.: C12Q 1/68, A61K 31/7088, A61K 31/713, C12N 15/113, A61P 25/04

(54) **INHIBITORS OF NOX4 EXPRESSION AND/OR NOX4 FUNCTION AND THEIR USE IN THE PREVENTION AND TREATMENT OF NEUROPATHIC PAIN**
HEMMER DER NOX4-EXPRESSION UND/ODER DER NOX4-FUNKTION UND IHRE VERWENDUNG BEI DER PRÄVENTION UND BEHANDLUNG VON NEUROPATHISCHEM SCHMERZ
INHIBITEURS DE L'EXPRESSION DE NOX4 ET/OU DE LA FONCTION DE NOX4 ET LEUR UTILISATION POUR LA PRÉVENTION ET LE TRAITEMENT DE LA DOULEUR NEUROPATHIQUE

(30) Priority: 15.09.2011 GB 201115992
(43) Date of publication of application: 06.08.2014
(73) Proprietor: Fraunhofer-Gesellschaft zur Förderung der angewandten Forschung e.V., 80686 München (DE)
(72) Inventor: SCHMIDTKO, Achim, 60316 Frankfurt (DE); KALLENBORN-GERHARDT, Wiebke, 65510 Wallrabenstein (DE); GEISSLINGER, Gerd, 65812 Bad Soden (DE); BRANDES, Ralf, 60596 Frankfurt (DE); SCHRÖDER, Katrin, 99441 Großschwabhausen (DE)
(74) Representative: Krauss, Jan
(86) International application number: PCT/EP2012/003857
(87) International publication number: WO 2013/037499

(56) References cited:
- WO-A1-2011/057171
- WO-A1-2011/072091
- WO-A2-2005/100392
- KALLENBORN-GERHARDT W ET AL: "The role NADPH Oxidase 4 derived reactive oxygen species in pain signaling", NAUNYN-SCHMIEDEBERG'S ARCHIVES OF PHARMACOLOGY, vol. 383, no. Suppl. 1, March 2011 (2011-03), page 29, XP009165268, & 77TH ANNUAL MEETING ON GERMAN-SOCIETY-FOR-EXPERIMENTAL-AND-CLINIC AL-PHARMACOLOGY-AND-TOXICOLOGY; FRANKFURT, GERMANY; MARCH 30 -APRIL 01, 2011
- Y.B. Im et al.: "miR23b ameliorates neuropathic pain in spinal cord by silencing NADPH oxidase 4", Antioxidants & redox signaling , 15 May 2012 (2012-05-15), XP007921335, Retrieved from the Internet: URL:http://www.nextbio.com [retrieved on 2012-11-28]
- KALLENBORN-GERHARDT W ET AL: "NADPH Oxidase 4 essentially contributes to nociceptive processing in neuropathic pain states", NAUNYN-SCHMIEDEBERG'S ARCHIVES OF PHARMACOLOGY, vol. 385, no. Suppl. 1, March 2012 (2012-03), page 43, XP009165270, & 78TH ANNUAL CONGRESS OF THE GERMAN-SOCIETY-FOR-EXPERIMENTAL-AND-CLINIC AL-PHARMACOLOGY-AND-TOXICOLOGY; DRESDEN, GERMANY; MARCH 19 -22, 2012
- KALLENBORN-GERHARDT WIEBKE ET AL: "NADPH oxidase-4 maintains neuropathic pain after peripheral nerve injury.", THE JOURNAL OF NEUROSCIENCE : THE OFFICIAL JOURNAL OF THE SOCIETY FOR NEUROSCIENCE 25 JUL 2012, vol. 32, no. 30, 25 July 2012 (2012-07-25) , pages 10136-10145, XP009165263, ISSN: 1529-2401
- SARAH GARRIDO-URBANI ET AL: "Targeting Vascular NADPH Oxidase 1 Blocks Tumor Angiogenesis through a PPAR[alpha] Mediated Mechanism", PLOS ONE, vol. 6, no. 2, 1 January 2011 (2011-01-01) , pages e14665-e14665, XP55041892, ISSN: 1932-6203, DOI: 10.1371/journal.pone.0014665
- M. SEDEEK ET AL: "Critical role of Nox4-based NADPH oxidase in glucose-induced oxidative stress in the kidney: implications in type 2 diabetic nephropathy", AJP: RENAL PHYSIOLOGY, vol. 299, no. 6, 1 December 2010 (2010-12-01), pages F1348-F1358, XP55041888, ISSN: 0363-6127, DOI: 10.1152/ajprenal.00028.2010
- ZIMMERMANN M: "Pathobiology of neuropathic pain", EUROPEAN JOURNAL OF PHARMACOLOGY, ELSEVIER SCIENCE, NL, vol. 429, no. 1-3, 1 January 2001 (2001-01-01), pages 23-37, XP008133255, ISSN: 0014-2999
- BARON RALF: "Neuropathic pain: a clinical perspective.", HANDBOOK OF EXPERIMENTAL PHARMACOLOGY 2009, no. 194, 2009, pages 3-30, XP009165261, ISSN: 0171-2004
- Y B IM ET AL: "Molecular targeting of NOX4 for neuropathic pain after traumatic injury of the spinal cord", CELL DEATH AND DISEASE, vol. 3, no. 11, 15 November 2012 (2012-11-15), page e426, XP55045947, DOI: 10.1038/cddis.2012.168

## Description

### Field of the invention

The present invention relates to modulators, in particular inhibitors, of the expression and/or the function of NADPH Oxidase 4 (Nox4) for use in the prevention and/or treatment of nerve injury, in particular pain, more particularly neuropathic pain. Further disclosed is a method for the identification of Nox4 modulators, a pharmaceutical composition comprising a Nox4 inhibitor and a method for preventing and treating pain, in particular neuropathic pain, in a subject in need of such a treatment. Also, the invention relates to inhibitors, of the expression and/or the function of NADPH Oxidase 4 (Nox4) for use in the prevention and/or treatment of neuropathic pain.

### Background of the invention

In general, myelination is one of the fundamental biological processes that forms segmented sheaths around large-calibre axons to provide insulation and protection of neurons. Myelination is a complex process, involving reciprocal neuron-glia interactions. Destruction or changes of myelination have been considered a causative event in numerous neurological diseases such as multiple sclerosis (Wang et al., 2011). In response to peripheral nerve injury, Schwann cells are activated and their myelin properties are modified, resulting in altered conduction properties of nociceptive fibres (Devor, 2006). In particular, the myelin degradation of Aβ afferents promotes susceptibility of their axonal plasma membrane to pronociceptive stimuli, leading to ectopic depolarization and mechanical allodynia (Kobayashi et al., 2008). Accordingly, neuropathic pain behaviours occurred after experimental demyelination of peripheral or central neurons (Wallace et al., 2003; Inoue et al., 2004; Moalem-Taylor et al., 2007; Ahn et al., 2009; Olechowski et al., 2009) and in mutant mice with aberrant myelination or loss of myelin (Gillespie et al., 2000; Chen et al., 2006). Moreover, neuropathic pain accompanies many demyelinating human diseases, such as Guillain-Barre syndrome, Charcot-Marie-Tooth type I disease, and multiple sclerosis (Ueda, 2008).

Pain in response to tissue damage and inflammation (inflammatory pain) or lesions to the peripheral or central nervous system (neuropathic pain) is characterized by a sensitization of the nociceptive system. This sensitization clinically manifests as pain in response to normally innocuous stimuli (allodynia), increased response to noxious stimuli (hyperalgesia) or spontaneous pain in the absence of a stimulus. Moreover, it can persist long after the initial injury is resolved, thereby causing major health problems (Dray and Read, 2007; Dray, 2008).

Various signalling cascades in primary afferent neurons and the spinal cord have been identified that mediate the pain sensitization. Recent studies suggest that reactive oxygen species (ROS) such as superoxide (O₂⁻) and hydrogen peroxide (H₂O₂) essentially contribute to the sensitization during persistent pain (Lee et al., 2010). Accordingly, pain was inhibited in animal models after systemic or intrathecal administration of free radical scavengers and superoxide dismutase mimetics (Kim et al., 2004; Hacimuftuoglu et al., 2006; Khattab, 2006; Gao et al., 2007; Lee et al., 2007; Schwartz et al., 2008; Kim et al., 2009; Tanabe et al., 2009). However, the sources of ROS production and the mechanisms by which ROS contribute to pain sensitization remain poorly understood.

NADPH oxidases of the Nox family are a group of enzymes whose sole known function is the production of ROS by catalysing electron transfer from NADPH to molecular O₂. Four rodent genes of the catalytic subunit Nox (Nox1-4) have been identified, each with tissue-specific expression and different functions in intracellular signalling (Lambeth, 2004; Brown and Griendling, 2009; Zhang et al., 2010).

Recent studies revealed that mice lacking Nox1 or Nox2 (also known as gp91ₚₕₒₓ) develop reduced nociceptive behaviours in animal models of persistent pain (Ibi et al., 2008; Kim et al., 2010), indicating a contribution of Nox-mediated O₂⁻ production to pain sensitization.

Nox1 mRNA was detected at high levels in the mouse paw and mice lacking Nox1 demonstrated a reduced nociceptive behaviour in inflammatory pain models (Ibi et al., 2008). Nox2 is rapidly induced in spinal cord microglia cells after peripheral nerve injury, and it contributes to the induction of injury-induced microglia activation, proinflammatory cytokine expression and neuropathic pain (Kim et al., 2010). However, these results have not been confirmed; according to Berger et al. (2011), Nox2 may only be a trigger of inflammatory and nitroxidative environment. So far, no reliable target for the preparation of a medicament to prevent and/or treat neuropathic pain has been found.

EP 2 002 835 discloses pyrazolo pyridine derivatives useful for the preparation of a pharmaceutical formulation for the modulation, notably the inhibition of the activity or function of the Nicotinamide adenine dinucleotide phosphate oxidase (NADPH Oxidase), whereby NADPH Oxidase is, among others, described as being involved in hyperalgesia associated with inflammatory pain.

US2011/0016541 discloses genetically modified animals and cells comprising edited chromosomal sequences encoding sensory-related proteins that are associated with nociception or taste disorders. Also provided are methods of using the genetically modified animals or cells disclosed herein to screen agents for toxicity and other effects. In particular, Cannabinoid receptor type 1 (CNR1) and type 2 (CNR2) are suggested to be useful for the treatment of inflammation and pain and have been investigated particularly for forms of pain that do not respond well to conventional treatments, such as neuropathic pain. Also, protein kinase A (PKA) is associated with hyperalgesia, and is indirectly associated with the regulation of phospho-CREB, a protein associated with hyperalgesia and neuropathic pain mechanisms. Further, LGALS1 (lectin galactoside-binding soluble 1), is associated with the potentiation of neuropathic pain in the dorsal horn.

Current favoured treatments of pain, in particular neuropathic pain, are antidepressants, e.g. serotonin-norepinephrine uptake inhibitors (SNRIs), strong opioid analgesics, gabapentin, pregabalin, or tramadol. However, many pharmacologic treatments decrease the sensitivity of nociceptive receptors or desensitize C fibres. SNRIs provoke an elevation of serotonin levels, loss of appetite, loss of sleep, drowsiness or headache. Also, strong opioid analgesics or weak ones such as tramadol are often not recommended as first line treatments. Conclusively, there is a strong need for alternative substances for use in the treatment of nerve injuries, pain, and neuropathic pain.

WO 2011/057171 discloses a method of treating or preventing neuroptahic pain by administering an siRNA targeting RhoA or TLR4.

### Summary of the invention

In view of the prior art described above, and the limitations of preventive or curative strategies currently available for nerve injuries, in particular pain, more particularly neuropathic pain, the object to be solved is to provide therapeutic strategies to prevent and/or treat neuropathic pain, by providing a target which plays an important role in the development of neuropathic pain, and to provide a method for the identification of substances that can be used as medicaments to prevent and/or treat neuropathic pain. Another object to be solved is to provide substances for the prevention and/or treatment of neuropathic pain

The object is solved by a method for identifying an inhibitor of NADPH-Oxidase 4 (Nox4) function and/or expression, comprising the steps of (a) contacting a biological sample, comprising a nucleic acid sequence encoding for nox4, or the gene expression product of Nox4, with a candidate compound; (b) assessing at least one of Nox4 activity or expression; and (c) comparing the activity or expression in step (b) with the activity or expression of Nox4 in the absence of the candidate compound, wherein a decrease between the measured activities or expression of Nox4 in step (b) compared to step (c) indicates that the candidate compound is an inhibitor of Nox4, and wherein a decrease between the measured activities or expression of Nox4 indicates that the candidate compound is for use in the prevention, treatment, of neuropathic pain.

Preferably, the decrease between the measured activities or expression of Nox4 in step (b) compared to step (c) indicates that the candidate compound is an inhibitor of Nox4, and is for use in the prevention and/or treatment of a nerve injury, preferably pain, more preferably neuropathic pain, in a subject in need thereof. In one embodiment, the biological sample of such method is contacted with the candidate compound *in vitro* or *in vivo.* In a further embodiment, the candidate compound is a protein, a peptide, a polypeptide, a polynucleotide, an oligonucleotide, or a chemical compound. In a preferred embodiment, the chemical compound is a pyrazolo pyridine derivative, or a pyrazolo piperidine derivative.

In yet a further embodiment of the method as described above, assessing the activity of Nox4 comprises an enzyme activity assay, immunoassay, or Western blotting. In one embodiment, assessing the expression of Nox4 comprises Northern blotting, microarray analysis, or RT-PCR. In one embodiment, assessing the expression of Nox4 comprises the assessment of reactive oxygen species (ROS) formation, preferably using techniques such as Amplex Red and dichlorodihydrofluorescein diacetate (DCFH-DA), preferably in cultured human umbilical vein endothelial cells (HUVEC) and/or neuroblastoma cells.

In a further embodiment, assessing the activity of Nox4 comprises assessing expression or activity of a gene regulated by Nox4, preferably the genes for matrix metalloproteinase-2 (MMP2), MAP kinase phosphatase-1 (MKP-1), p38, MAP kinase or serum response factor (SRF), or any gene regulated by, or regulating, by said genes. Assessing the activity of an enzyme or assessing the expression of a gene comprises all methods for assessing the activity of an enzyme known to the skilled person.

The disclosure also relates to a method for the preparation of a pharmaceutical composition for use in the prevention and/or treatment of a nerve injury in a subject in need thereof, comprising the steps of: (a) identifying a modulator according to the method as described above, wherein the modulator is an inhibitor of NADPH-Oxidase 4 (Nox4) function and/or expression; and (b) preparing a pharmaceutical formulation comprising the inhibitor of step (a), and, optionally, a pharmaceutical acceptable carrier.

In one aspect of the disclosure the nerve injury is pain, preferably neuropathic pain. In a further embodiment, the nerve injury, pain, or neuropathic pain is associated with dysmyelination. In a preferred embodiment, the pain is neuropathic pain. In another aspect, the nerve injury, or pain, or neuropathic pain is associated with dysmyelination. In another aspect, dysmyelination is associated with leukodystrophies, schizophrenia, or multiple sclerosis. In yet another aspect, the leukodystrophy can be Peliazaeus-Merzbacher disease, Canavan disease, or phenylketonurie. In yet another aspect, the nerve injury or neuropathic pain is associated with Guillan-Barre syndrome, Charcot-Marie Tooth type I disease, and/or multiple sclerosis.

The object is further solved by an inhibitor, of NADPH-Oxidase 4 (Nox4) expression and/or function as defined in the claims for use in the prevention and/or treatment of pain in a mammal. Preferably, the inhibitor is for use in the prevention and/or treatment of neuropathic pain, in a mammal. In a preferred embodiment, the mammal is a human.

In one aspect of the disclosure, the modulator, or inhibitor, or candidate compound, is a chemical compound. In a further aspect, the chemical compound is a pyrazolo pyridine derivative. In a further aspect, the chemical compound is a pyrazolo piperidine derivative. In one embodiment, the inhibitor of Nox4 is selected from antisense DNA- and/or RNA-oligonucleotides, antisense 2'-O-methyl oligoribonucleotides, antisense oligonucleotides containing phosphorothioate linkages, small-interfering RNA, miRNA, antisense oligonucleotides containing Locked Nucleic Acid LNA® bases, morpholino antisense oligos, PPAR-gamma agonists, antagomirs, and mixtures thereof, and in particular an antagomir of Nox4.

The object is further solved by a pharmaceutical composition comprising an inhibitor of Nox4 as defined in the claims for use in the prevention and/or treatment of neuropathic pain. In one embodiment, the pharmaceutical composition comprising an inhibitor of Nox4 is for use in the prevention and/or treatment of neuropathic pain. In another embodiment, the nerve injury, or pain, or neuropathic pain is associated with dysmyelination. In another embodiment, dysmyelination is associated with leukodystrophies, schizophrenia, or multiple sclerosis. In yet another embodiment, the leukodystrophy can be Peliazaeus-Merzbacher disease, Canavan disease, or phenylketonurie. In yet another embodiment, the nerve injury or neuropathic pain is associated with Guillan-Barre syndrome, Charcot-Marie Tooth type I disease, and/or multiple sclerosis. A pharmaceutical composition can also comprise an activator of Nox4.

In yet another embodiment, the inhibitor of Nox4, or the pharmaceutical composition as described above, can be administered orally, rectally, transmucosally, transdermally, intestinally, parenterally, intramuscularly, intrathecally, direct intraventricularly, intravenously, intraperitoneally, intranasally, intraocularly, or subcutaneously.

The disclosure further relates to a method of treating or preventing a nerve injury, preferably pain, more preferably neuropathic pain, in a subject, comprising the step of administering an effective amount of an NADPH Oxidase inhibitor (Nox4). In another aspect of the disclosure, the nerve injury, or pain, or neuropathic pain is associated with dysmyelination. In another aspect, dysmyelination is associated with leukodystrophies, schizophrenia, or multiple sclerosis. In yet another aspect, the leukodystrophy can be Peliazaeus-Merzbacher disease, Canavan disease, or phenylketonurie. In yet another aspect, the nerve injury or neuropathic pain is associated with Guillan-Barre syndrome, Charcot-Marie Tooth type I disease, and/or multiple sclerosis.

"Pain" includes, without being limited to it, nociceptive pain, neuropathic pain, psychogenic pain, and pain due to functional disorders. Nociceptive pain is caused by stimulation of peripheral nerve fibres that respond only to stimuli approaching or exceeding harmful intensity (nociceptors), and includes, without limiting, thermal, mechanical, and chemical nociceptive pain, as well visceral, deep somatic, and superficial somatic pain. Nociceptive pain also includes inflammatory pain and spastic pain. The term "neuropathic pain" refers to pain due to irritation or damage or disorders of the peripheral or central nerve system, and results from lesions or diseases affecting the somatosensory system. "Neuropathic pain" includes peripheral neuropathic pain, central neuropathic pain, or mixed (peripheral and central) neuropathic pain. Nociceptive or neuropathic pain may be associated with allodynia, hyperalgesia, spontaneous pain, and dysesthesia.

The nerve injury, or pain, or neuropathic pain can, without being limited to it, be associated with dysmyelination. Dysmylination can be associated with leukodystrophies, schizophrenia, or multiple sclerosis. A leukodystrophy can be Peliazaeus-Merzbacher disease, Canavan disease, or phenylketonurie. The nerve injury, pain, or neuropathic pain, can be associated with Guillan-Barre syndrome, Charcot-Marie Tooth type I disease, and/or multiple sclerosis.

"Dysmyelination" is characterized by a defective structure and function of myelin sheaths. Human diseases where dysmyelination has been implicated include, without being limited to it, leukodystrophies (Pelizaeus-Merzbacher disease, Canavan disease, phenylketonuria) and schizophrenia.

A "modulator" of Nox4 function and/or expression is a substance that regulates or changes the function and/or expression of Nox4. Such modulator may directly or indirectly influence Nox4 function and/or expression. Nox4 function and/or expression can be changed or regulated by, for example, and without being limited to it, binding to a domain of the Nox4 protein, or enhancing or suppressing gene expression of Nox4. A modulator according to the disclosure may also indirectly regulate or change the function and/or expression of Nox4 by regulating or changing the function and/or expression of a gene that regulates, or is regulated, by Nox4.

A "biological sample" is a specimen of any biological source. The biological source can be any naturally occurring or genetically modified organism. The biological sample can derive from, without being limited to it, tissues, cell cultures, crude extracts, body fluids, as well as from solutions of gene expression products, nucleotide acids, proteins, or peptides, or nucleotide acids, proteins, or peptides as solid matter. "Gene expression products" according to the invention comprise, but are not limited to, purified, recombinant, natural, artificial or synthetic nucleotide sequences, like DNA, cDNA, RNA, or mRNA; or proteins, or peptides. "Gene expression products" referred to herein comprise, but are not limited to, purified, recombinant, natural, artificial or synthetic gene expression products, or modifications thereof. A nucleic acid sequence referred to herein is any nucleic acid sequence encoding for nox4 known in prior art, or complementary sequences, or nucleotide sequences hybridizing thereto under stringent conditions, as well as modifications thereof.

An "inhibitor" is a substance that can reduce the effectiveness of a catalyst in a catalysed reaction (either a non-biological catalyst or an enzyme). An inhibitor referred to herein can reduce the effectiveness of the activity of an enzyme; also, an inhibitor referred to herein can reduce the effectiveness of the expression of an enzyme. An inhibitor may be, without being limited to it, recombinant, natural, artificial or synthetic nucleotide sequences, like DNA, cDNA, RNA, or mRNA; or proteins, or peptides, or modifications thereof. An inhibitor may be, without being limited to it, any nucleic acid sequence, or complementary sequences, or nucleotide sequences hybridizing under stringent conditions to a nucleotide sequence encoding for nox4 known in prior art, as well as modifications thereof.

A "cell" according to the invention can be a prokaryotic or eukaryotic cell. A "cell" according to the invention is preferably, and without being limited to it, selected from neuronal cells, or other cells derived from mammalian neuronal cells, such as dorsal root ganglia neurons, neuroblastoma cell lines (N1E, NB41A3, Neuro2A, CHP212, IMR32 or NG108), medulloblastoma cell lines (D341 or D283), PC12 cells or Schwann cells from peripheral nerves, cultured human umbilical vein endothelial cells (HUVEC) and/or neuroblastoma cells which in a further preferred embodiment recombinantly, or inherently, express, or preferably overexpress, Nox genes. The mammalian cells may be preferably selected from rabbit, mouse or rat, preferably rat dorsal root ganglia or neuroblastoma cells. Preferably, the cell is a mouse cell. The term "cell" also includes cells of an animal model. Also, a cell can be part of a tissue culture.

The term "prevention" in the context of the present invention shall be understood as a medical intervention which aims to avoid the occurrence of a negative event which most likely leads to the worsening of the condition of a patient having a disease, or to the injury or the death of a healthy and/or ill subject.

A "subject in need thereof" can be, without being limited to it, any animal or human suffering of pain, especially neuropathic pain. Preferably, the subject in need thereof is a human.

The invention is based on the finding that the ROS-producing NADPH oxidase isoform Nox4 is expressed in a subset of non-peptidergic nociceptors and myelinated dorsal root ganglia neurons. Mice lacking Nox4 demonstrated normal responses in animal models of acute or inflammatory pain. However, their late-phase neuropathic pain behaviour after peripheral nerve injury was substantially reduced. Moreover, persisting neuropathic pain behaviour was inhibited after tamoxifen-induced deletion of Nox4 in adult Nox4-CreERT2 transgenic mice. Comparison of whole-genome expression profiles after peripheral nerve injury revealed that the loss of Nox4 markedly attenuated injury-induced dysmyelination processes of peripheral nerves. The results show that ROS derived from Nox4 essentially contribute to nociceptive processing in neuropathic pain states. Accordingly, inhibition of Nox4 provides a novel therapeutic modality for the treatment of neuropathic pain.

A method for identifying substances that inhibit NADPH-Oxidase 4 (Nox4) thus contributes to find substances and formulations of pharmaceutical compositions that are useful for the prevention and the treatment of a nerve injury, particularly pain, more particularly neuropathic pain that can be associated with dysmyelination.

### Detailed description of the invention

The invention is based on the finding that Nox4 is predominately expressed in IB4-binding nociceptors and their central terminals in the spinal cord. This subset of non-peptidergic primary afferent neurons plays a particular role in the sensitization of pain pathways (Braz et al., 2005; Basbaum et al., 2009). Nox4 consequently contributes to nociceptive processing. The nox4 gene encodes a member of the NOX-family of enzymes that functions as the catalytic subunit the NADPH oxidase complex. The encoded protein is localized to non-phagocytic cells where it catalyzes the reduction of molecular oxygen to various reactive oxygen species (ROS). Nox4 has been implicated in numerous biological functions including signal transduction.

Regulation, or modulation, of Nox4 can thus influence pain perception in a subject. An activator of Nox4 function and/or expression may thus enhance pain perception in a patient with analgesia. Inhibition of Nox4 can thus inhibit nociceptive processing and, as a result, prevent or treat pain. An inhibitor may reduce the activity or expression of Nox4, thus assessing activity or expression of Nox4 in cell being contacted with a substance can be used to identify an inhibitor of Nox4. A method for identifying a modulator, in particular an inhibitor, of NADPH-Oxidase 4 (Nox4) according to the invention is thus suitable to identify substances that are useful in the regulation, prevention and/or the treatment of a nerve injury, in particular pain.

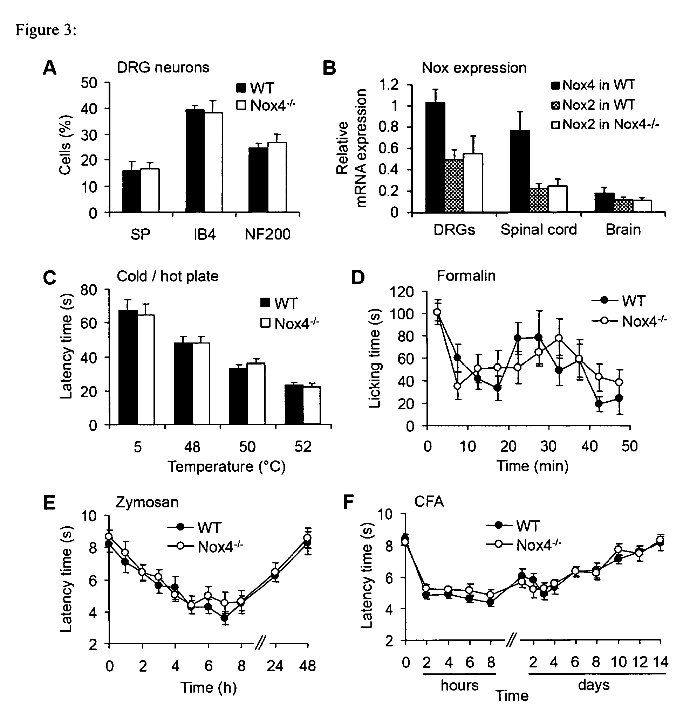

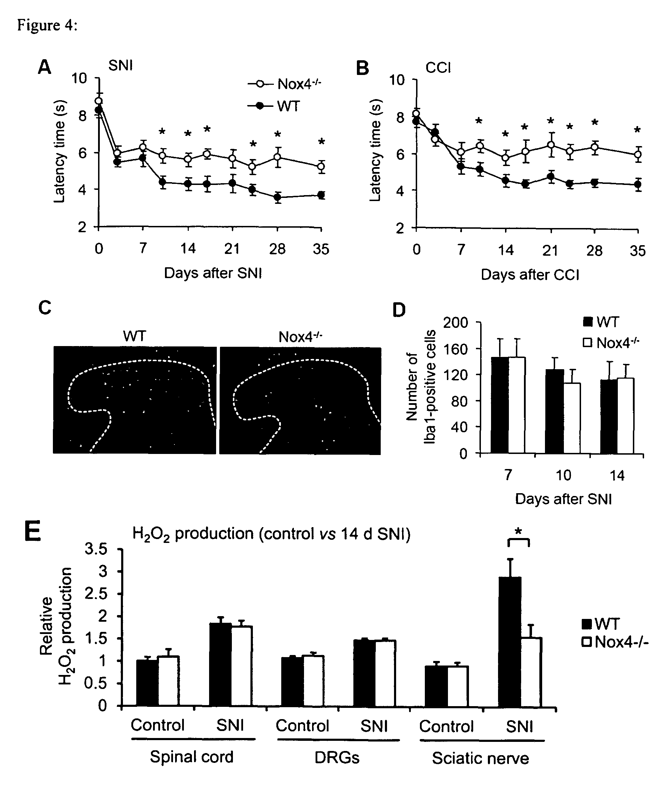

Expression of Nox2 in DRGs, the spinal cord and brain is not altered in Nox4^{-/-} mice (Fig. 3B). Nox1 and Nox3 mRNA cannot reliably be detected in both Nox4^{-/-} and wild-type (WT) mice, showing that other Nox enzymes are not compensatory upregulated due to the lack of Nox4. After peripheral nerve injury induced by SNI or CCI, the extent of mechanical allodynia was significantly reduced in Nox4^{-/-} mice as compared to WT mice (Fig 4A). During the first 7 days after SNI or CCI surgery, mechanical allodynia was indistinguishable in both groups. However, at later stages mechanical allodynia was considerably reduced in Nox4^{-/-} mice compared with WT mice (Fig. 4B). Conclusively, Nox4-derived ROS essentially contribute to the maintenance of neuropathic pain after peripheral traumatic axonal injury.

The neuropathic pain behaviour was reduced not only in Nox4^{-/-} mice but also in tamoxifen-inducible Nox4-CreERT2 mice that were treated with tamoxifen after the SNI-induced mechanical allodynia was fully developed. This observation rules out several potentially confounding factors such as developmental defects or compensatory mechanisms that could have complicated the interpretation of the reduced neuropathic pain behaviour in Nox4^{-/-} mice. In addition, these results show that reducing Nox4 expression, or presumably selective inhibition of Nox4 activity, is beneficial in neuropathic pain states that already persist for a longer time. A mild tamoxifen treatment protocol (1 mg/d i.p. for 3 d) was used to avoid possible undesired tamoxifen-induced side effects that might impair the analysis of nociceptive behaviour. The tamoxifen treatment led to a significant reduction of Nox4 mRNA to ~ 50% of baseline levels in DRGs but not in the spinal cord at 21 d after treatment. This shows that tamoxifen and its metabolite 4-hydroxytamoxifen, which induces the Credependent recombination *in vivo,* do not easily cross the blood-brain barrier, thus limiting recombination in the spinal cord. However, the fact that the neuropathic pain behaviour was significantly attenuated in these mice shows that Nox4 expressed in primary afferent neurons rather than in the spinal cord exerts the 'pain-relevant' effects after peripheral nerve injury.

Accordingly, the method for identifying a modulator, in particular an inhibitor, of NADPH-Oxidase 4 (Nox4) according to the invention is in particular suitable to identify substances that are useful in the prevention and/or the treatment of neuropathic pain.

Several lines of evidence are provided herewith that Nox4-derived ROS also play an important role in dysmyelination after peripheral nerve injury. First, western blot and immunohistochemical analyses demonstrate that the nerve injury-induced degradation of myelin-specific proteins were abolished in Nox4^{-/-} mice. Second, morphological investigations demonstrates a decreased number of enlarged fibres with thin myelin sheaths and of myelin in-/outfoldings in injured sciatic nerves of Nox4^{-/-} mice. Finally, but importantly, a whole-genome expression analysis revealed that the nerve injury-induced upregulation of several genes coding for myelin-specific proteins, which most likely reflects a compensatory mechanism in response to the protein degradation, did not occur in Nox4^{-/-} mice. Hence, ROS derived from Nox4 mediate the degradation of myelin components in peripheral nerves after injury, and the resulting dysmyelination contributes to injury induced neuropathic pain.

Accordingly, the method for identifying an inhibitor of NADPH-Oxidase 4 (Nox4) according to the invention is in particular suitable to identify substances that are useful in the prevention and/or the treatment of a nerve injury, pain, or neuropathic pain associated with dysmyelination.

The method can be *performed in vivo* or *in vitro,* whereby an *in vivo* method also comprises the use of an animal model. A mouse model is used in the Examples disclosed herein. The advantages of an *in vitro* model is easy handling and reduced costs. The Nox4 inhibitory activity can be measured *in vitro* by assessment of reactive oxygen species (ROS) formation using techniques such as Amplex Red and dichlorodihydrofluorescein diacetate (DCFH-DA) in cultured human umbilical vein endothelial cells (HUVEC) and neuroblastoma cells. The candidate compound is a chemical compound, a protein, a peptide, or a polypeptide, or a polynucleotide, or an oligonucleotide, inhibiting the expression of Nox4. Antisense DNA-and/or RNA-oligonucleotides, antisense 2'-O-methyl oligoribonucleotides, antisense oligonucleotides containing phosphorothioate linkages, antisense oligonucleotides containing Locked Nucleic Acid LNA® bases, small interfering RNAs, miRNAs, morpholino antisense oligos, antagomirs, and mixtures thereof. These are effective means for the inhibition of the expression of specified targets, and may in particular be acting as inhibitor of the Nox4 protein or inhibiting the expression of Nox4.

Also, a chemical substance can be screened in the method of the invention, as chemical compounds are very useful in the formulation of pharmaceutical compositions. EP 2 002 835 discloses pyrazolo pyridine derivates and EP 2 361 912 discloses pyrazolo piperidine derivatives for the inhibition of NADPH oxidases, thus the compounds disclosed therein are preferred substances to be screened, and preferred inhibitors.

Methods for assessing the enzyme activity of an enzyme are known to the skilled person. Especially Western blot, 3D electrophoresis, enzyme activity electrophoresis, and enzyme activity assays in a vessel are reliable methods.

There are multiple techniques for the identification and quantification of nucleic acids, in particular RNA, known in the state of the art. Besides Northern blotting and RT-PCR, assessing RNA expression can be performed by means of RNA expression arrays, fluorescent nucleic acid probes, for example coupled to membranes or beads, and antibody based detection systems. In an indirect approach, the activity of the RNA is further measured by *in vitro* or *in vivo* reporter assays. For example, the person of skill in the art could use without harnessing inventive skill design reporter assays based on sequence of Nox4 that allow for an easy screening of candidate inhibitors. The target-sequence of the RNA can be introduced into the 3' or 5' untranslated regions of a reporter gene of choice. Such construct can be transformed into a suitable cell expression system, which is subsequently brought into contact with the candidate compound. The activity of the reporter gene in samples that were contacted with the compound in comparison with the activity of the reporter gene in control samples gives information about the inhibitory effect of the tested compound.

Genes regulated by Nox4 are, for example, the genes for matrix metalloproteinase-2 (MMP2), MAP kinase phosphatase-1 (MKP-1), p38 MAP kinase or serum response factor (SRF) (Brown and Griendling, 2009).

Accordingly, as an alternative, enzyme or expression activity of Nox4 can be assessed indirectly by assessing enzyme or expression activity of enzymes that are regulated by Nox4. Such enzymes are matrix metalloproteinase-2 (MMP2), MAP kinase phosphatase-1 (MKP-1), p38 MAP kinase or serum response factor (SRF). Further methods according to the invention are microarray assays, as well as any method suitable for the detection of gene expression, or enzyme activity, known to the skilled person.

Interestingly, recent studies suggest that Nox4 activity can be modified by posttranslational mechanisms (Lambeth et al., 2007). For example, insulin or lipopolysaccharide activate Nox4-dependent ROS generation within minutes (Mahadev et al., 2004; Park et al., 2004), a response that is too rapid to be accounted for by increased Nox4 protein expression. Moreover, Nox4 activation independent from its transcriptional upregulation has been observed in angiotensin II-stimulated mesangial cells (Gorin et al., 2003) and in PMA-stimulated vascular endothelial cells (Kuroda et al., 2005), and recent data indicate that polymerase delta interacting protein 2 (Lyle et al., 2009), oxidized phospholipids (Lee et al., 2009) and tyrosine kinase substrate with 5 SH3 domains (Diaz et al., 2009) may increase Nox4 activity. Accordingly, enzymes involved in these mechanisms are preferred candidate substrates or inhibitors.

A method for the preparation of a pharmaceutical composition for use in the prevention and/or treatment of a nerve injury in a subject in need thereof, comprising the steps of: (a) identifying a modulator according to the screening method of the invention, wherein the modulator is an inhibitor of NADPH-Oxidase 4 (Nox4) function and/or expression; and (b) preparing a pharmaceutical formulation comprising the inhibitor of step (a), and, optionally, a pharmaceutical acceptable carrier provides suitable medicaments for the treatment of said nerve injuries, or, in the case of the modulation of Nox4 with an activator, suitable medicaments for the treatment and/or regulation of pain perception.

While it is possible that, for use in therapy, an inhibitor of Nox4 expression and/or function may be administered as the raw chemical, it is preferable to present the active ingredient as a pharmaceutical composition. The carrier and/or excipient of the pharmaceutical composition must be "acceptable" in the sense of being compatible with the other ingredients of the formulation and not deleterious to the recipient thereof.

Inhibitors or pharmaceuticals of the invention provide an alternative medication for a nerve injury, on particular pain, or neuropathic pain, and may avoid side effects of the medication of the state of the art.

With Nox4 being target for the prevention and/or treatment of pain, in particular neuropathic pain, a method of treatment for pain is possible by using Nox4 inhibitors, in particular by using Nox4 inhibitors that were identified by the method of the invention. Such method of treatment may avoid the side effects of medicaments of the state of the art.

The invention shall be described in more detail on the basis of the following examples and with reference to the attached Figures, without being limited thereto.

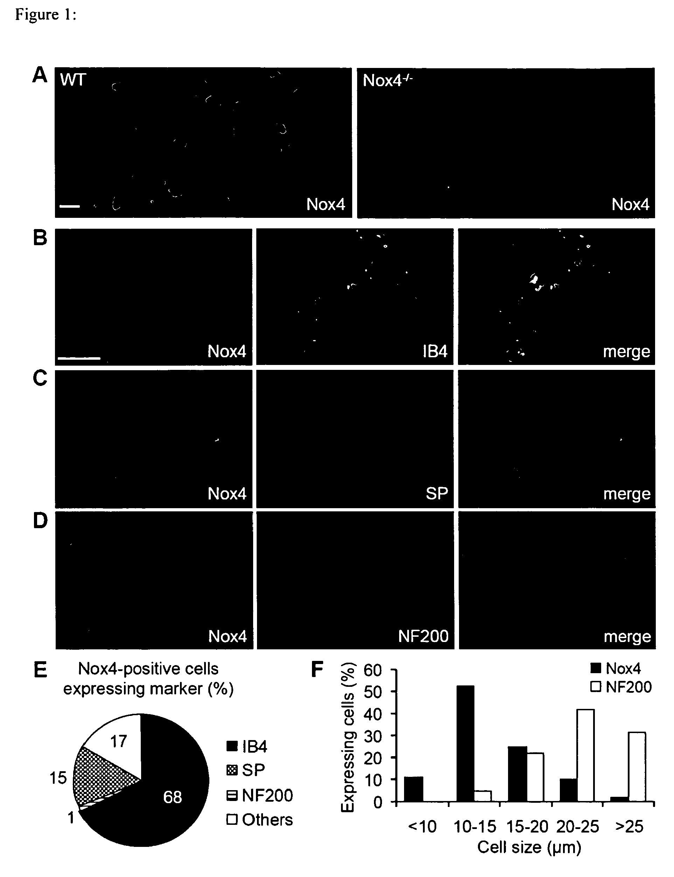

**Figure 1****.** Expression of Nox4 in DRGs. ***A*,** Immunohistochemistry of Nox4 in lumbar DRGs of wild-type mice and Nox4^{-/-} mice reveals specific Nox4 expression in 12.8% ± 0.8% of DRG neurons (4456 cells counted). ***B-D,*** Typical examples of Nox4 immunoreactivity in subpopulations of DRG neurons labelled using IB4 binding or using antibodies to substance P (SP) or NF200, respectively. ***E***, Quantitative summary of DRG cell populations expressing Nox4 protein from experiments represented in ***B-D.*** Most Nox4-expressing DRG neurons bind IB4 (68%) and are therefore unmyelinated and non-peptidergic, whereas Nox4 almost never colocalizes with the SP-containing peptidergic subpopulation of unmyelinated neurons. A few Nox4-expressing cells coexpress NF200 (15%), indicating that they are myelinated DRG neurons. ***F*,** Size distribution of Nox4-expressing cells compared to those expressing NF200 demonstrating that Nox4 is mainly expressed in DRG neurons of small- and medium diameter. Scale bars, 25 µm.

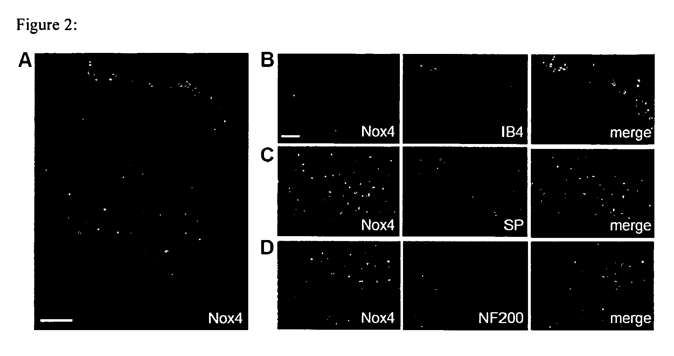

**Figure 2****.** Expression of Nox4 in the spinal cord. ***A,*** Immunostaining shows Nox4 predominately in the dorsal horn of the spinal cord. Scale bar, 50 µm. ***B-D,*** Nox4 immunofluorescence in the dorsal horn (laminae I-III) overlaps to a large extent with binding to IB4 (B) but little if at all with immunoreactivity of substance P (C) or neurofilament 200 (D). Scale bar, 20 µm.
**Figure 3****.** Basal characteristics, acute pain behaviour and inflammatory pain behaviour are not impaired in Nox4^{-/-} mice. ***A*,** Percentages of DRG neurons binding IB4 or immunoreactive for SP or NF200 were similar in WT and Nox4^{-/-} mice (3340 cells counted). ***B,*** Expression of Nox mRNA in DRGs, spinal cord and brain of WT and Nox4^{-/-} mice assessed by quantitative real-time RT-PCR. Nox4 mRNA was only detected in tissues from WT mice, whereas Nox2 mRNA levels were similar in tissues from WT and Nox4^{-/-} mice. Nox1 or Nox3 mRNA were not detected (*n* = 3-4). *C*, Cold and hot plate tests. The latency of Nox4^{-/-} mice to exhibit nocifensive behaviours is similar to that of WT littermates at cold (5 °C) and hot (48-52 °C) temperatures (*n* = 12). ***D*,** Formalin test. Both genotypes show a similar biphasic response to 5% formalin injected into a hindpaw (*n* = 8). ***E***, ***F***, Paw withdrawal latency times after mechanical stimulation after injection of zymosan (E) or CFA (F) into a hindpaw. Mechanical hyperalgesia did not differ between genotypes at all times tested (*n* = 7-9). All data are presented as mean ± SEM.
**Figure 4****.** Reduced neuropathic pain behaviour in Nox4^{-/-} mice. ***A**, **B**,* Paw withdrawal latency times of Nox4^{-/-} and WT mice after mechanical stimulation in the spared nerve injury (SNI, *A*) and chronic constriction injury (CCI, B) models of neuropathic pain. Nox4^{-/-} mice demonstrate reduced mechanical allodynia as compared to WT littermates during 10 to 35 days after SNI or CCI (*n* = 12-14). ***C, D,*** Ibal immunoreactivity in the spinal cord of Nox4^{-/-} and WT mice after SNI indicates that microglia activation induced by peripheral nerve injury is not impaired in Nox4^{-/-} mice (*n* = 4 mice per group). Representative examples of Ibal immunofluorescence (red) in the spinal cord 14 d after SNI are presented in C, dotted lines delineate grey matter. Quantitative analysis of Ibal-positive cells in the dorsal horn 7-14 d after SNI are shown in *D.* All data are presented as mean ± SEM. ** p* < 0.05, comparing Nox4^{-/-} and WT mice. *E*, H₂O₂ production in the spinal cord, DRGs, and the sciatic nerve of control animals and day 14 SNI animals. The SNI-induced H₂O₂ production was significantly lower in the sciatic nerve of Nox4^{-/-} mice compared with WT mice. Data are presented as mean ± SEM. *p<0.05, comparing Nox4^{-/-} and WT mice.

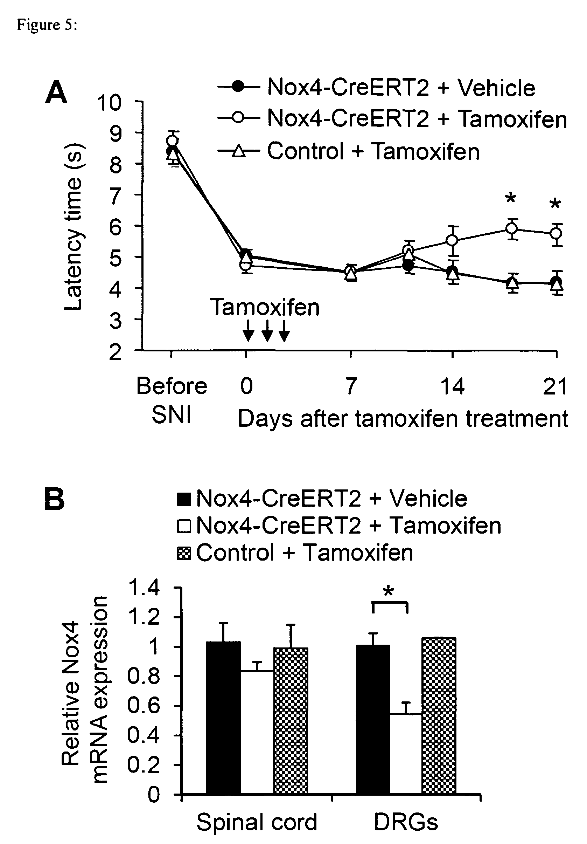

**Figure 5****.** Reduced neuropathic pain behaviour after inducible deletion of Nox4. ***A***, Paw withdrawal latency times after mechanical stimulation in tamoxifen-inducible Nox4-CreERT2 mice and littermate control mice during SNI-induced neuropathic pain. Tamoxifen (1 mg/d) or vehicle was i.p. injected on 3 consecutive days starting 10 d after SNI surgery (indicated by arrows). Mechanical allodynia was gradually reduced in tamoxifen-treated Nox4-CreERT2 mice in contrast to tamoxifen-treated control mice or vehicle-treated Nox4-CreERT2 mice (n = 8-10). ***B,*** Nox4 mRNA expression in DRGs and the spinal cord at the end of the observation period (i.e. 21 d after the first tamoxifen injection) assessed by quantitative real-time RT-PCR. Tamoxifen treatment significantly reduced Nox4 mRNA levels in DRGs of Nox4-CreERT2 mice as evaluated by quantitative real-time RT-PCR.

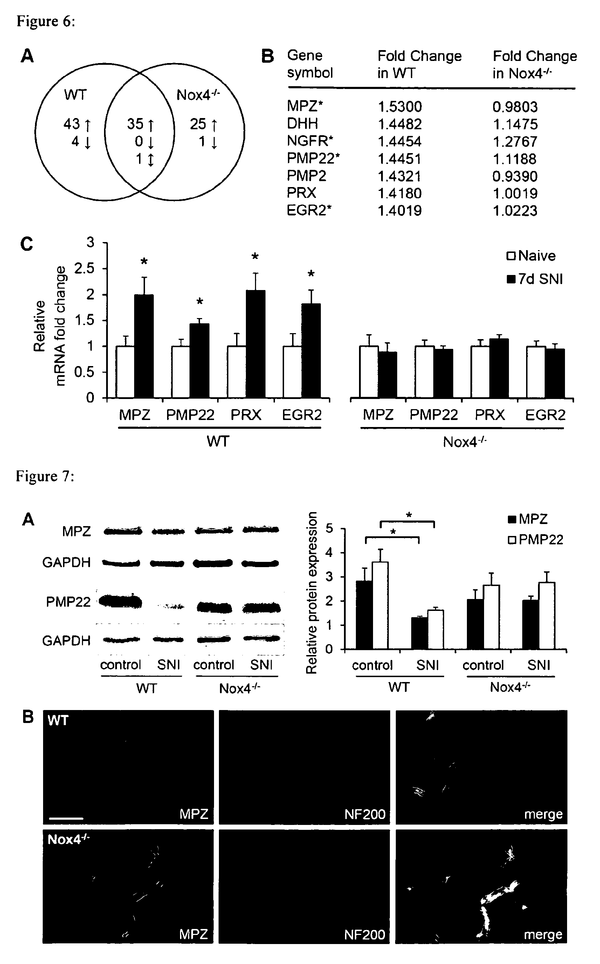

**Figure 6****.** Gene expression profiles in the spinal cord of Nox4^{-/-} and WT mice after SNI. ***A**,* Diagram of the numbers of regulated genes 7 d after SNI as compared to naive animals identified by the whole-genome microarray screen (*p* < 0.05 and fold change > 1.4 or < 0.6). ***B,*** Molecules associated with myelination processes that are upregulated (> 1.4 fold) in WT but not in Nox4^{-/-} mice after SNI. * Molecules identified by Ingenuity® pathway analysis. ***C*,** Real-time RT-PCR of selected transcripts confirms that myelination-associated molecules are upregulated after SNI only in WT mice. n = 3 mice per group. Data are presented as mean ± SEM. * *p* < 0.05, comparing SNI-treated and naive mice.
**Figure 7****.** Expression of myelin-specific proteins in the sciatic nerve of WT and Nox4^{-/-} mice after SNI. ***A*,** Western blot analysis of the myelin-specific proteins MPZ and PMP22 in the day 14 SNI sciatic nerve and the uninjured control sciatic nerve. GAPDH was used as loading control. Note that MPZ and PMP22 protein expression is significantly decreased after SNI in WT mice but not in Nox4^{-/-} mice. *n* = 3 mice per group. Data are presented as mean ± SEM. * *p <* 0.05, comparing control and SNI sciatic nerves. ***B, C***, Immunostaining of the day 14 SNI sciatic nerve shows increased MPZ (B) and PMP22 (C) immunoreactivity in Nox4^{-/-} mice as compared to WT mice, whereas immunoreactivity of the neuronal marker NF200 is similar in both genotypes. Scale bar, 10 µm.

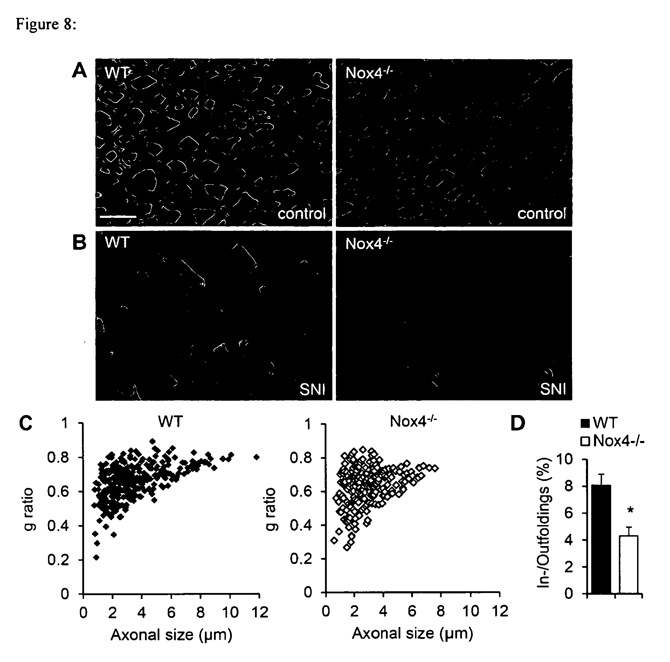

**Figure 8****.** Structural alterations in the sciatic nerve of WT and Nox4^{-/-} mice after SNI. ***A**,* Toluidine blue-stained semithin cross sections of sciatic nerves of naive mice demonstrate normal appearance of myelinated fibres in WT and Nox4^{-/-} mice. Scale bar, 5 µm. ***B,*** Toluidine blue stainings in proximal segments 1 mm from the injured site 14 d after SNI show marked alterations in the sciatic nerve of WT and Nox4^{-/-} mice including reduced axonal density, enlarged axons and myelin infoldings and outfoldings. ***C*,** Scatter plots display *g* ratios of individual fibres (obtained from three animals per group) as a function of the respective axon diameter (*n* = 348 and 243 fibres for WT and Nox4^{-/-} mice, respectively). Note that the proportion of enlarged axons with thin myelin sheaths (expressed by a higher g ratio) is reduced in Nox4^{-/-} mice. ***D*,** The percentage of myelin infoldings and outfoldings in the day 14 SNI sciatic nerve is significantly reduced in Nox4^{-/-} mice. Data are presented as mean ± SEM. ** p <* 0.05.

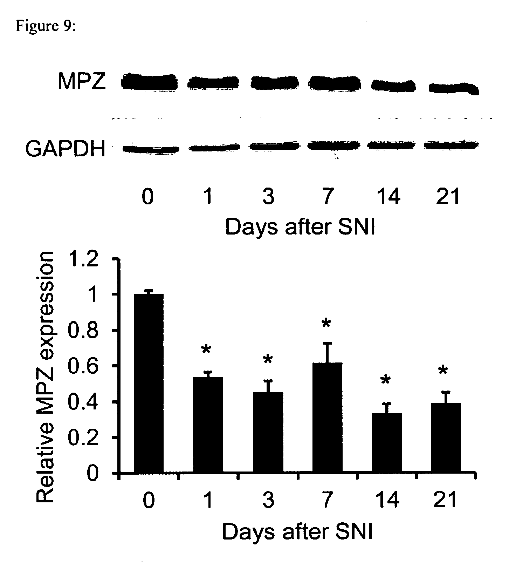

**Figure 9****.** MPZ expression in the sciatic nerve after SNI. Western blot analysis of the myelin specific protein MPZ in the proximal sciatic nerve stump of C57BL/6 mice revealed that MPZ protein expression is significantly decreased from 1 to 21 d after SNI. GAPDH was used as loading control. *n* = 3 mice per group. Data are presented as mean ± SEM (*p<0.05).

### EXAMPLE 1: Materials and Methods

### Animals

The generation of mice lacking Nox4 (Nox4^{-/-} mice) has been described elsewhere (Zhang et al., 2010). Experiments were performed in 6- to 12-week-old mice of either sex backcrossed onto C57BL/6 background. Tamoxifen-inducible Nox4 knock-out mice (Nox4-CreERT2 mice) were produced by crossing homozygous Nox4-floxed mice and heterozygous tamoxifen-inducible CreERT2 mice in which the Cre recombinase, fused to a mutated estrogen ligand-binding domain (ER^{T2}) that requires the presence of tamoxifen for activity (Indra et al., 1999), is under the control of the ubiquitous ROSA26 promoter. C57BL/6N mice (Harlan) were additionally used for RT-PCR analyses. Animals were housed on a 12/12 h light/dark cycle with standard rodent chow and water available *ad libitum.* All experiments were approved by the local Ethics Committee for Animal Research.

### Behavioural tests

Littermate mice were used in all behavioural studies. All investigations were done by a blinded observer.

*Rotarod test.* Motor coordination was assessed with a Rotarod treadmill for mice (Ugo Basile) at a constant rotating speed of 32 rpm. All mice had five training sessions before the day of the experiment. The fall-off latency was averaged from five tests and the cutoff time was 120 s.

*Cold- and hot-plate test.* Mice were placed on a cooled or heated metal plate surrounded by a Plexiglas cylinder (Hot/Cold Plate; Ugo Basile). The time between placement and shaking or licking of a hindpaw was recorded. Temperatures of 5, 48, 50 and 52 °C were tested with cutoff times of 90, 80, 60 and 40 s, respectively, to prevent tissue damage. Only one test per animal was performed because repeated measures might cause profound latency changes (Mogil et al., 1999).

*Formalin test.* A 5% formaldehyde solution (15 µl; formalin) was injected subcutaneously into the dorsal site of one hindpaw (Hunskaar et al., 1985). The time spent licking the formalin-injected paw was recorded in 5 min intervals up to 50 min after formalin injection.

*Mechanical paw sensitivity.* Paw withdrawal latency after mechanical stimulation was measured with an automated von Frey-type testing device (Dynamic Plantar Aesthesiometer; Ugo Basile) which allows for reliable detection of mechanical sensitivity in mice (Schmidtko et al., 2008). The stainless steel probe of the touch stimulator unit was pushed against the paw with ascending force until a strong and immediate withdrawal occurred. The maximum force was set at 5 g and the ramp speed was 0.5 g/s. The paw withdrawal latency was calculated as the mean of 4-6 measurements with at least 20 s in between.

*Zymosan- or CFA-induced hyperalgesia.* Fifteen microliter of a zymosan A suspension (5 mg/ml in 0.1 M PBS, pH 7.4; Sigma-Aldrich) or 20 µl of complete Freund's adjuvant (CFA; containing 1 mg/ml heat-killed Mycobacterium tuberculosis in paraffin oil 85% and mannide monooleate 15%; Sigma-Aldrich) was injected into the plantar subcutaneous space of a hindpaw (Meller and Gebhardt 1997).

*Neuropathic pain.* The "spared nerve injury" (SNI) model and the "chronic constriction injury" (CCI) model were used to investigate neuropathic pain behavior. Surgery was carried out under isoflurane anaesthesia. For SNI, two branches of the sciatic nerve were ligated and cut distally, leaving the sural nerve intact (Decosterd and Woolf, 2000). For CCI, three silk ligatures (6-0) were tied around the proximal sciatic nerve, thereby constricting the nerve by about 30-50% (Bennett and Xie, 1988).

*Tamoxifen induction.* Tamoxifen (Sigma Aldrich) was prepared by dissolving in ethanol (10 mg per 100 µl) and mixing this solution with 900 µl corn oil for a final concentration of 10 mg/ml. Eight- to ten-week-old Nox4-CreERT2 mice were i.p. injected with 100 µl tamoxifen solution (1 mg, corresponding to about 40 mg/kg tamoxifen) once a day at 10, 11 and 12 d after SNI.

### Immunohistochemistry

Mice were intracardially perfused with 0.9% saline followed by 4% paraformaldehyde in PBS (pH 7.4), under deep isoflurane anaesthesia. The lumbar spinal cord (L4-L5), DRGs (L4-L5) and sciatic nerve were dissected, post-fixed for 10 min in the same fixative and cryoprotected in 20% sucrose overnight. Tissues were frozen in tissue freezing medium (Leica) on dry ice, cryostat-sectioned at a thickness of 14-16 µm and stored at -80°C. For immunofluorescence, sections were permeabilized for 5 minutes in PBST (0.1% Triton®-X in PBS), blocked for 1 h using 10% normal goat or donkey serum and 3% bovine serum albumin (BSA) in PBS, and incubated with primary antibodies diluted in 3% BSA in PBST over night at 4°C or for 2 h at room temperature. The following antibodies were used: rabbit anti-Nox4 (1:800; kindly provided by Ajay M. Shah (Anilkumar et al., 2008)), rat anti-substance P (1:200; BD Biosciences), mouse anti-NF200 (clone N52; 1:1000; Sigma Aldrich), rabbit anti-Ibal (1:500; Wako), chicken anti-MPZ (1:500; Neuromics) and rabbit anti-PMP22 (1:200; Abcam). Slides were then washed in PBS and stained with secondary antibodies conjugated with Alexa® Fluor 488 (Invitrogen) or Cy3 (Sigma-Aldrich). Alexa® Fluor 488-conjugated Griffonia simplicifolia isolectin B4 (IB4; 10 µg/ml in PBS; Invitrogen) was incubated for 2 h at room temperature. In double-labeling experiments, primary antibodies were consecutively incubated. Images were taken using an Axio® Observer.Z1 microscope (Zeiss) equipped with a monochrome CCD camera (AxioCam® Mrm; Zeiss). Images were taken with different filters, pseudocoloured and superimposed using the Zeiss AxioVision® 4.7.2 software. Adjustment of brightness and contrast was done using Adobe Photoshop® CS software. Controls were performed by omitting the first and/or the second primary antibodies and by incubating tissues of Nox4^{-/-} mice. DRG cell size analyses were performed using ImageJ software (NIH).

### Microglia quantification

Microglia activation after SNI was investigated by an observer blinded to the animal genotype using Ibal immunohistochemistry on sections of 16 µm thickness as described above. To find the precise localization of the area affected by the SNI surgery, Alexa® Fluor 488-conjugated IB4 was additionally incubated on the slides together with the Cy3-conjugated secondary antibody. In the area affected by SNI surgery the IB4 staining pattern in lamina II fades (Casals-Diaz et al., 2009). Spinal cord sections at a distance of about 300 µm (7-9 sections per mouse, 4 mice per genotype) were stained and images of the dorsal horn (lamina I-IV) were captured under a 10× objective. Only slides in which the IB4 staining was interrupted were analyzed. Using ImageJ software equipped with the MacBiophotonics MBF plugin (McMaster Biophotonics Facility, McMaster University, Hamilton, ON), Ibal image contrast was adjusted such that the background level just disappeared, and the same cutoff level was used for all images (command: image > adjust > colour threshold). Images were then converted into 8-bit (command: image > type > 8-bit), the cell bodies were selected according to their size using nucleus counter plugin (command: plugins > particle analysis > nucleus counter; smallest particle size: 50; threshold method: otsu), and Ibal-positive cell bodies were counted. Similar results were obtained in control experiments using manual counting of Ibalpositive cell bodies.

### Morphological analysis of sciatic nerves

Under deep isoflurane anaesthesia, mice were perfused with 0.9% saline followed by a solution containing 4% PFA and 0.5% glutaraldehyde in PBS (pH 7.4). Sciatic nerves were dissected, post-fixed in the same fixative overnight and embedded in Epon using standard procedures. Control and SNI-operated nerves of Nox4^{-/-} and WT mice were cross-sectioned at a thickness of 1 µm and stained with toluidine blue. The morphology of injured nerves was analyzed at a distance of 1 mm proximal to the lesion. The number of myelinated axons as well as the number of in- and outfoldings of the myelin sheath were counted. Axonal diameter was measured using ImageJ software equipped with the MacBiophotonics MBF plugin. Images were converted into 8-bit (command: image > type > 8-bit) and the image threshold was adjusted so that the background level (not-myelinated axons and other cells) disappeared. The diameter of the areas surrounded by the myelin sheath was measured (command: analyze > set measurements > feret's diameter; command: analyze > analyze particles: Size: 50 - Infinity, Circularity: 0.0 - 1.0, Show: Overlay Masks; Settings: Display results, Clear results, Summarize, Exclude on edges, In situ Show) and the mean value of the smallest (MiniFeret) and longest diameter (Feret) of the area surrounded by the myelin was calculated and taken as the axon diameter. Myelin thickness was measured using the line selection tool at three individual points of each axon and the mean value was taken for further analysis. Using a scale bar, measured values were converted into µm and the g ratio was calculated. A total number of 857 myelinated axons were analysed.

### Western Blot

Ipsilateral and contralateral sciatic nerves from SNI operated mice were rapidly dissected, frozen in liquid nitrogen and stored at -80°C until use. Samples were homogenized in Phosphosafe buffer (Novagen) combined with a protease inhibitor cocktail (Complete Mini; Roche Diagnostics) and centrifuged at 14000 x g for 1 h. Extracted proteins (20 µg per lane) were separated by SDS-polyacrylamid gel electorphoresis and blotted onto a nitrocellulose membrane. Membranes were blocked in blocking buffer (Odyssey® blocking buffer, LI-COR Bioscience; diluted 1:1 in PBS) for 1 h at RT and then incubated with rabbit anti-MPZ (1:500; Neuromics), rabbit anti-PMP22 (1:500; Abcam) or mouse anti-GAPDH (1:2000; Ambion) dissolved in blocking buffer containing 0.1% Tween®-20 over night at 4°C. After incubation with secondary antibodies for 1 h at RT, proteins were detected using an Odyssey® Infrared Imaging System (LI-COR Bioscience). Quantification of band densities was done using ImageJ software.

### Real-time RT-PCR

Mice were exsanguinated under deep isoflurane anaesthesia, and tissues were dissected, snap frozen in liquid nitrogen and stored at -80 °C. Total RNA from spinal cord, brain and DRGs was extracted under RNase-free conditions using a RNA isolation kit (for spinal cord and brain: RNeasy® Lipid Tissue Mini Kit; Qiagen; for DRGs: RNAqueous Micro Kit; Ambion) according to the manufacturer's instructions, DNase treated for 15 min to minimize genomic DNA contamination and quantified with a NanoDrop® ND-1000 spectrophotometer (NanoDrop® Technologies). cDNA was synthesized using 200 ng RNA, random hexamer primers, RT-Enhancer and the Verso®-enzyme of the Verso® Kit (Thermo Scientific). Real-time RT-PCR was performed with an ABI Prism® 7500 Sequence Detection System (Applied Biosystems) using TaqMan Gene Expression Assays for murine Nox4 (Mm01317086_ml), Nox1 (Mm00549170_ml), Nox2 (Mm01287742_ml), Nox3 (Mm01339132_ml), GAPDH (Mm99999915_gl) and β-actin (Mm00607939_sl), purchased from Applied Biosystems. Reactions (total volume, 10 µl) were performed in duplicate or triplicate by incubating at 95 °C for 10 min, followed by 40 cycles of 15 s at 95 °C and 1 min at 60 °C. Water controls were included to ensure specificity. Relative expression of target gene levels was determined using the comparative ΔΔCt method, with Ct indicating the cycle number at which the signal of the PCR product crosses an arbitrary threshold set within the exponential phase of the PCR. The amount of sample RNA was normalized to GAPDH. Control experiments revealed similar results if β-actin was used for normalization.

### Microarray analysis

*RNA Isolation and Analysis.* Lumbar (L4-L5) spinal cords of littermate Nox4^{-/-} and WT mice (naive and 7 d after SNI, 3 animals per group) were dissected and frozen in liquid nitrogen. Total RNA was isolated using the RNeasy® Lipid Tissue Mini Kit (Qiagen) according to the manufacture's instructions. Full microarray service, including labelling and hybridization, was provided by the German Cancer Research Center (DKFZ, Heidelberg). The quality of total RNA was checked by gel analysis using the total RNA Nano chip assay on an Agilent® 2100 Bioanalyzer. Only samples with RNA index values greater than 8.5 were selected for expression profiling. RNA concentrations were determined using a NanoDrop® spectrophotometer.

*Probe Labelling and Illumina*® *Sentrix*® *BeadChip array Hybridization.* Biotin-labelled cRNA samples for hybridization on Illumina® Mouse Sentrix-6® BeadChip arrays were prepared according to Illumina®'s recommended sample labelling procedure based on the modified Eberwine protocol (Eberwine et al., 1992). In brief, 250-500 ng total RNA was used for cDNA synthesis, followed by in vitro transcription to synthesize biotin-labelled cRNA using a MessageAmp® II cRNA Amplification kit (Ambion). Biotin-16-UTP was purchased from Roche Applied Science. The cRNA was column purified using a TotalPrep RNA Amplification Kit and eluted in 60-80 µl water. Quality of cRNA was controlled using the RNA Nano Chip Assay on an Agilent® 2100 Bioanalyzer and spectrophotometrically quantified (NanoDrop®).

Hybridization was performed at 58 °C in GEX-HCB buffer (Illumina®) at a concentration of 100 ng cRNA/µl and unsealed in a wet chamber for 20 h. Spike-in controls for low, medium and highly abundant RNAs were added, as well as mismatch control and biotinylation control oligonucleotides. Microarrays were washed once in High Temp Wash buffer (Illumina®) at 55 °C and then twice in E1BC buffer (Illumina®) at room temperature for 5 min (in between washed with ethanol at room temperature). After blocking for 5 min in 4 ml of 1% (wt/vol) Blocker Casein in PBS Hammarsten grade (Pierce Biotechnology), array signals were developed by incubation in 2 ml of 1 µg/ml Cy3-streptavidin (Amersham Biosciences, Buckinghamshire, UK) solution and 1% blocking solution for 10 min. After a final wash in E1BC the arrays were dried and scanned.

*Scanning and data analysis.* Microarray scanning was done using a BeadStation array scanner, setting adjusted to a scaling factor of 1 and PMT settings at 430. Data extraction was done for all beads individually, and outliers > 2.5 MAD (median absolute deviation) were removed. All remaining data points were used for the calculation of the mean average signal for a given probe, and standard deviation for each probe was calculated. Data analysis was done by normalization of the signals using the quantile normalization algorithm without background subtraction, and differentially regulated genes were defined by calculating the standard deviation differences of a given probe in one-by-one comparisons of samples or groups.

### Statistical analysis

Statistical analysis was performed with SPSS software using the Student's t test for paired comparisons, or one-way ANOVA for multiple comparisons followed by a Fisher post hoc test. When mice were tested at different time points a repeated-measures ANOVA were used and differences between groups at each time point were analysed with a Fisher post-hoc test. Rotarod fall-off latencies were analysed with Mann-Whitney U-test and are expressed as median and interquartile range. All other data are presented as the mean ± standard error of the mean (SEM). For all tests, a probability value P < 0.05 was considered as statistically significant.

### EXAMPLE 2: Nox4 expression in dorsal root ganglia and the spinal cord

First, the Nox4 distribution in dorsal root ganglia (DRGs) by immunohistochemistry (Fig 1A) was examined and it was found that 13% of sensory neurons expressed Nox4. Specificity of the Nox4 antibody was confirmed by the absence of immunoreactivity in DRGs of Nox4^{-/-} mice m(Fig. 1A). The detailed distribution of Nox4 in DRGs was investigated by double-labelling immunohistochemistry experiments with established markers. Interestingly, 68% of Nox4-positive cells bound the lectin IB4, a marker of the non-peptidergic population of unmeyelinated nociceptors (Fig. 1B and 1E), whereas there was virtually no overlap of Nox4-positive cells with substance P, a marker of peptidergic unmyelinated nociceptors (Fig. 1C and 1E). In addition, 15% of Nox4-positive cells expressed NF200, a neurofilament marker of neurons with myelinated axons (Fig. 1D and 1E). Corresponding to the double labelling experiments, cell size analyses revealed that Nox4 was mostly expressed in small to medium diameter DRG neurons (Fig. 1F). Hence, the results show that Nox4 is preferentially expressed in non-peptidergic nociceptors and in some myelinated DRG neurons.

In the spinal cord, Nox4 immunoreactivity predominates in the superficial dorsal horn (Fig. 2A). Double-labelling experiments revealed a high extent of colocalisaion of Nox4 with IB4-binding terminals of primary afferent neurons in the inner part of lamina II (Fig. 2B), consistent with the observation that most Nox4-expressing DRG neurons bind IB4. Virtually no colocalization was observed between Nox4 and terminals of primary afferent neurons immunoreactive for substance P (Fig. 2C), whereas some Nox4 immunoreactivity was colocalized with NF200 immunoreactivity (Fig. 2D). Altogether, Nox4 is predominately expressed in IB4-binding nociceptors and their central terminals in the spinal cord. Because this subset of non-peptidergic primary afferent neurons plays a particular role in the sensitization of pain pathways (Braz et al., 2005; Basbaum et al., 2009), Nox4 contributes to nociceptive processing.

### EXAMPLE 3: Normal basal sensitivity and inflammatory pain behaviour in Nox4^{-/-} mice

To assess the role of Nox4 in nociceptive processing in vivo, the nociceptive behaviour of Nox4^{-/-} mice with that of littermate wild-type (WT) mice were compared in various animal models of pain. Nox4^{-/-} mice are viable and fertile, normal in size and do not display any gross physical or behavioural abnormalities (Zhang et al., 2010). The overall frequencies of DRG neuron populations positive for substance P, IB4, or NF200 were similar between WT and Nox4^{-/-} mice (Fig. 3A). The macroscopic morphology of DRGs and the spinal cord, and the distribution of terminals of nociceptive and thermoreceptive primary afferents in the superficial dorsal horn appeared normal in Nox4^{-/-} mice, showing that the lack of Nox4 did not affect the morphology or general structural properties of sensory neurons.

Quantitative real-time RT-PCR analyses revealed that expression of Nox2 in DRGs, the spinal cord and brain is not altered in Nox4^{-/-} mice (Fig. 3B). Nox1 and Nox3 mRNA were not reliably detected in both genotypes (CT values > 35), showing that other Nox enzymes are not compensatory upregulated due to the lack of Nox4. Furthermore, the motor coordination and balance is not impaired in Nox4^{-/-} mice, as analysed in the rotarod test (median fall-off latencies: Nox4^{-/-} mice, 103.4 sec [interquartile range 73.1 - 120.0 sec]; WT mice, 108.8 sec [interquartile range 78.2 - 120.0 sec]; P = 0.801; n = 11-12 per group).

To determine acute nociception in Nox4^{-/-} mice, the latency times to acute thermal stimuli using the cold-plate (5 °C) and hot-plate (48, 50 and 52 °C) test was measured. No significant differences in latency times were found between Nox4^{-/-} and WT mice (Fig. 3C), showing that the lack of Nox4 does not affect the immediate response to acute noxious thermal stimulation. In order to test whether Nox4 deficiency affects the rapid sensitization in pain pathways, we performed the 5% formalin test (Hunskaar et al., 1985). Intraplantar formalin evokes two phases of spontaneous pain-related behaviour, an immediate short-lasting first phase that results from nociceptor activation, and a second phase that involves a period of activity-dependent central sensitization (Coderre et al., 1990; Vardeh et al., 2009). No significant differences in formalin-induced pain sensitivity during both phases between Nox4^{-/-} mice and their control littermates were found (Fig. 3D). The sum of licking time in Nox4^{-/-} and WT mice were: phase 1, 136.3 ± 12.5 s and 161.5 ± 13.0 s (P = 0.184); phase 2, 436.4 ± 75.9 s and 381.6 ± 65.0 s (P = 0.592), respectively. Then, the extent of mechanical hyperalgesia evoked by injection of zymosan or CFA into a hindpaw was tested, two well-established models of inflammatory pain (Meller and Gebhart, 1997; Ferreira et al., 2001). After zymosan injection, Nox4^{-/-} mice developed mechanical hyperalgesia in the injected hindpaw that was indistinguishable from that of WT mice (Fig. 3E). Similarly to the zymosan model, there were no significant differences between Nox4^{-/-} and WT mice in the mechanical hyperalgesia induced by CFA injection (Fig. 3F). Moreover, the CFA-evoked paw oedema developed to an equivalent extent in both genotypes, as analysed 14 d after CFA injection.

### EXAMPLE 4: Reduced neuropathic pain behaviours in Nox4^{-/-} mice

To assess the role of Nox4-derived ROS in neuropathic pain, the behaviour of Nox4^{-/-} mice and littermate WT mice was examined in the spared nerve injury (SNI) model, which is characterized by injury-induced mechanical allodynia (Decosterd and Woolf, 2000). Mechanical allodynia of the affected hindpaw developed similarly in Nox4^{-/-} and WT mice during the first 7 d after nerve injury (Fig. 4A). Interestingly however, at later time points (i.e., between 10 d after nerve injury and the end of the 35 d observation period) the extent of mechanical allodynia was significantly reduced in Nox4^{-/-} mice as compared to WT mice (Fig 4A). Similar to the SNI model, mechanical allodynia was indistinguishable in both groups during the first 7 days after chronic constriction injury (CCI) surgery. However, at later stages mechanical allodynia was considerably reduced in Nox4^{-/-} mice compared with WT mice (Fig. 4B). Together, these data show that Nox4-derived ROS essentially contribute to the maintenance of neuropathic pain after peripheral traumatic axonal injury.

Previous studies demonstrated that spinal cord microglia activation contributes to the pathological hypersensitivity after peripheral nerve injury (Costigan et al., 2009). Because this microglia reaction was markedly reduced in Nox2-deficient mice (Kim et al., 2010), the SNI-induced microglia activation was assessed in Nox4^{-/-} mice. However, no differences in expression of the microglia marker Ibal in the spinal cord at 7, 10 and 14 d after SNI surgery were detected (Fig. 4C-D). These data show that ROS derived from Nox4, in contrast to those derived from Nox2, are not involved in nerve injury-induced microglia activation in the spinal cord. Furthermore, the data also indicate that Nox4 and Nox2 affect neuropathic pain signalling by different mechanisms.

### EXAMPLE 5: Inducible deletion of Nox4 attenuates neuropathic pain behaviour

Global gene ablation may cause developmental compensatory adaptations that might affect phenotypic changes in the genetically manipulated adult animal. To circumvent these issues, mice carrying a conditional null allele of Nox4 (Nox4^{fl/fl}) were crossed with tamoxifen-inducible ERT2Cre transgenic mice, with the resulting homozygous inducible conditional Nox4 knock-out mice (Nox4^{fl/fl};ERT2^{Cre}) referred to as Nox4-CreERT2 mice. The Nox4^{fl/fl} littermates were referred to as control mice. Animals were subjected to SNI surgery to induce mechanical allodynia. Ten days after SNI, mice were i.p. injected with tamoxifen (1 mg/d for 3 d) to activate Cre recombinase and knock-down Nox4, and paw withdrawal latency times were measured up to 21 d after the first injection. As shown in Fig. 5A, mechanical allodynia was not affected during the first 7 d after tamoxifen treatment. However, at later stages tamoxifen-treated Nox4-CreERT2 mice gradually recovered from mechanical allodynia, whereas in control mice mechanical allodynia remained nearly constant until the end of the 21 d observation period (Fig. 5A). Investigation of Nox4 expression levels in Nox4-CreERT2 mice 21 d after tamoxifen treatment revealed a decrease of Nox4 mRNA to 83% (P = 0.116) in the spinal cord and to 54% (P = 0.008) in DRGs as compared to vehicle-treated Nox4-CreERT2 mice (Fig. 5B). These data show that the tamoxifen treatment protocol was sufficient for significant knock-down of Nox4 in DRGs. In conclusion, neuropathic pain behaviour is attenuated by temporal somatic knock-down of Nox4 in adult mice.

### EXAMPLE 6: Gene expression profiles in Nox4^{-/-} and WT mice after SNI

To identify mechanisms that might account for the reduced neuropathic pain behaviour in Nox4^{-/-} mice the relative changes in mRNA expression in the spinal cord of Nox4^{-/-} and WT mice were analysed 7 d after SNI compared to naive control mice using a whole-genome expression analysis. Tissues from three mice per group were analysed to provide three independent biological replicates. Labeled RNAs were hybridized onto the Illumina® Mouse Sentrix-6® BeadChip array containing more than 46,000 genes, and were analysed using the Illumina® BeadStudio software. Regulated genes in SNI-treated mice were defined using criteria of p < 0.05 and an overall fold change from naive mice of > 1.4 for up-regulated genes and < 0.6 for down-regulated genes. A total of 109 genes were identified that were regulated by SNI in either WT or Nox4^{-/-} mice, or in both genotypes. Of these 109 genes, 35 were upregulated in both WT and Nox4^{-/-} mice, whereas one gene was decreased in WT mice and increased in Nox4^{-/-} mice. Forty-seven genes were changed uniquely in WT mice, whereas 26 genes were regulated only in Nox4^{-/-} mice. More genes were upregulated than downregulated in both genotypes (Fig. 6A).

Next, the regulated genes were analysed with Ingenuity® pathway analysis software (Ingenuity® Systems) to identify biological functions differentially regulated in Nox4^{-/-} mice as compared to WT mice. The top five overall physiological system functions in both genotypes are shown in Table 1:

**Table 1. Top five overall physiological functions of regulated genes in WT and Nox4^{-/-} mice using Ingenuity® pathway analysis**

| | *P* values | Genes |
|---|---|---|
| Functions regulated in WT mice | | |
| Nervous System Development and Function | 5.07 x 10⁻⁶ - 8.00 x 10⁻³ | 19 |
| Hematological System Development and Function | 6.13 x 10⁻⁶ - 8.00 x 10⁻³ | 22 |
| Tissue Morphology | 6.13 x 10⁻⁶ - 7.71 x 10⁻³ | 18 |
| Immune Cell Trafficking | 1.40 x 10⁻⁵ - 8.00 x 10⁻³ | 13 |
| Tissue Development | 5.09 x 10⁻⁵ - 8.00 x 10⁻³ | 18 |
| | | |
| Functions regulated in Nox4^{-/-} mice | | |
| Hematological System Development and Function | 2.23 x 10⁻⁶ - 1.22 x 10⁻² | 18 |
| Immune Cell Trafficking | 2.23 x 10⁻⁶ - 1.22 x 10⁻² | 12 |
| Tissue Development | 3.13 x 10⁻⁵ - 1.22 x 10⁻² | 17 |
| Hematopoiesis | 8.12 x 10⁻⁵ - 8.12 x 10⁻³ | 6 |
| Cardiovascular System Development and Function | 9.74 x 10⁻⁵ - 1.22 x 10⁻² | 6 |

In WT mice, the category most significantly regulated after SNI was "nervous system development and function". Interestingly, this category was not listed among the top five functions in Nox4^{-/-} mice. Therefore, the top annotations of the "nervous system development and function" category were analysed in WT mice (Table 2) and it was found that several molecules that are associated with myelination processes were significantly regulated in WT mice after SNI, including early growth response 2 (EGR2, also called Krox20), myelin protein zero (MPZ, also called P0), nerve growth factor receptor (NGFR) and peripheral myelin protein 22 (PMP22).

**Table 2. Top five annotations in Category "Nervous System Development and Function" in WT mice**

| Functions Annotation | *P* value | Genes |
|---|---|---|
| Myelination of neurons | 5.07 x 10⁻⁰⁶ | EGR2*, NGFR*, PMP22* |
| Myelination of cells | 9.05 x 10⁻⁰⁵ | EGR2*, MPZ*, NGFR*, PMP22* |
| Neurological process of neurons | 1.84 x 10⁻⁰⁴ | CCL13*, EGR2*, GAL, MPZ*, NGFR*, NPY, PMP22*, XDH* |
| Neurological process of nerves | 2.08 x 10⁻⁰⁴ | EGR2*, MPZ*, NGFR* |
| Synaptic transmission of neurons | 2.71 x 10⁻⁰⁴ | CCL13*, GAL, MPZ*, NPY, PMP22*, XDH* |

| | | |
|---|---|---|
| * Regulated only in WT but not in Nox4^{-/-} mice. CCL13, chemokine (C-C motif) ligand 13; EGR2, early growth response 2; GAL, galanin prepropeptide; MPZ, myelin protein zero; NGFR, nerve growth factor receptor; NPY, neuropeptide Y; PMP22, peripheral myelin protein 22; XDH, xanthine dehydrogenase. | | |

Of note, the expression of these genes was not regulated after SNI in Nox4^{-/-} mice. Moreover, among the 109 regulated genes additional molecules that are associated with myelination processes but were not linked with myelination in the Ingenuity® pathway analysis software were identified, including desert hedgehog (DHH) (Sharghi-Namini et al., 2006), periaxin (PRX) (Marchesi et al., 2010) and peripheral myelin protein 2 (PMP2), which encodes the myelin P2 protein (Majava et al., 2010). Again, these genes were upregulated in WT mice but not in Nox4^{-/-} mice after SNI (Fig. 6B). The expression profiles of four of these myelination-associated genes were tested by quantitative real-time RT-PCR and it was confirmed that their expression was significantly upregulated after SNI in WT mice but not in Nox4^{-/-} mice (Fig. 6C). Altogether, these results show that myelination dependent processes induced by peripheral nerve injury is differentially regulated in WT and Nox4^{-/-} mice.

### SNI-induced ROS production in peripheral nerves is reduced in Nox4^{-/-} mice

To determine the sites of Nox4-induced ROS production after peripheral nerve injury, the inventors measured H₂O₂ levels in the spinal cord, DRGs, and the sciatic nerve (proximal nerve stump) of WT and Nox4^{-/-} mice using the Amplex Red assay. The inventors compared H₂O₂ levels in naive control animals and in animals 14 d after SNI (i.e., at a time point of reduced hypersensitivity in Nox4^{-/-} mice). In control animals, H₂O₂ was detected to a similar extent in WT and Nox4^{-/-} mice, indicating a limited contribution of Nox4 to basal H₂O₂ production in the investigated tissues (Fig. 4*E*). After SNI, H₂O₂ levels were increased in all investigated tissues of both genotypes. Notably, in the sciatic nerve, the SNI induced H₂O₂ production was significantly lower in Nox4^{-/-} mice compared with WT mice, whereas in the spinal cord and in DRGs, it was comparable between genotypes (Fig. 4E). These data demonstrate that H₂O₂ production is considerably increased in peripheral nerves after injury and that injury-induced H₂O₂ production in peripheral nerves mostly depends on Nox4. Furthermore, the data point to a limited contribution of Nox4 to SNI-induced H₂O₂ production in DRGs and the spinal cord.

### EXAMPLE 7: SNI-induced nerve fibre dysmyelination is reduced in Nox4^{-/-} mice

Injury of peripheral nerves results in marked changes in myelination of the proximal nerve stump, characterized by swollen myelin, altered myelin thickness and degradation of myelin components (Nagai et al., 2010). The aberrant myelination is accompanied by spontaneous action potentials in primary afferent nerves and sensitization of sensory processing, thereby contributing to injury-induced hyperalgesia and allodynia (Wallace et al., 2003; Kobayashi et al., 2008). To monitor the time course of injury-induced dysmyelination of peripheral nerves in the SNI model, the protein levels of MPZ, the main peripheral myelin protein, in C57BL/6 mice during 21 d after SNI were analyzed. As shown in Figure 9, SNI induced a significant decrease of MPZ expression in the proximal sciatic nerve stump during the entire observation period. These data indicate that a persistent dysmyelination occurs in the proximal sciatic nerve stump after SNI.

The observed differences in injury-induced regulation of myelination-associated genes in Nox4^{-/-} and WT mice prompted to further explore the myelination status of sciatic nerves in both genotypes 14 d after SNI, i.e. at a time point of reduced allodynia in Nox4^{-/-} mice (see Fig. 4A). For that purpose, the protein levels of MPZ, the main peripheral myelin protein, and PMP22, another major myelin component in the peripheral nervous system were analysed. In WT mice, the protein levels of MPZ and PMP22 were significantly reduced in the day 14 SNI sciatic nerve as compared to the uninjured sciatic nerve (Fig. 7A).

The protein expression of MPZ and PMP22 in injured nerves dropped by 53.9% and 55.4%, respectively, showing profound SNI-induced changes in the composition of peripheral myelin. Notably, a decrease in MPZ or PMP22 protein expression in injured sciatic nerves did not occur in Nox4^{-/-} mice (Fig. 7A), which parallels the observation that myelination-associated genes are regulated after nerve injury in WT but not in Nox4^{-/-} mice.

We further assessed the MPZ localization in the day 14 SNI sciatic nerve by immunohistochemistry on longitudinal sections. As shown in Fig. 7B, in both genotypes most MPZ immunoreactivity was colocalized with the neurofilament marker NF200, which reflects the presence of MPZ in myelin sheaths surrounding axons of primary afferent neurons.

However, the MPZ immunofluorescence intensity was considerably reduced in nerves of WT mice as compared to those of Nox4^{-/-} mice (Fig. 7B), confirming the differences in MPZ protein levels detected by western blot analyses. Similar differences in immunofluorescence intensities between WT and Nox4^{-/-} mice were observed in immunohistochemical stainings using the PMP22 antibody (data not shown). Together, these results show that peripheral nerve injury causes a drop of MPZ and PMP22 protein levels in the myelin sheaths surrounding the axons of injured nerves, and that Nox4 is essential for this effect.

Then, histological examinations of sciatic nerves on semithin cross sections stained with toluidine blue were performed. Figure 8A depicts a normal appearance of the sciatic nerve from naive WT and Nox4^{-/-} mice, with small and large diameter myelinated fibres regularly distributed and axonal diameters of no more than 5.6 µm in both genotypes. Two weeks after SNI, the proximal segments of injured nerves displayed severe fibre dystrophy and a decrease of axon density (number of axons per mm²) in all animals (Fig. 8B), consistent with previous reports (Inoue et al., 2004; Nagai et al., 2010). To estimate the myelination status, the axon (inner myelin sheath circle) and myelin diameters in the day 14 SNI sciatic nerve were measured using NIH ImageJ software and the g ratio, i.e. the numerical ratio between axon and fibre diameter was calculated. In both genotypes, lowest g ratios were detected in small diameter myelinated fibres (Fig. 8C). This observation shows that small diameter axons were encapsulated by myelin sheaths with a higher relative thickness (indicated by a lower g ratio), which was similar between WT and Nox4^{-/-} mice. In contrast, a decrease of relative myelin thickness (indicated by a higher g ratio) occurred with increasing axon diameter (Fig. 8C).

Interestingly, axons with diameters larger than the maximum axonal diameter in naive mice (5.6 µm) were detected after nerve injury. Of note, the proportion of these enlarged axons with relatively thin myelin sheaths was decreased in Nox4^{-/-} mice as compared to WT mice (6.2% versus 12.9%, respectively), showing that Nox4 contributes to the injury-induced processing of dysmyelination of large diameter fibres. Finally, the injury-induced myelin infoldings and outfoldings were assessed (see Fig. 8B), which predominately affect large diameter fibres and are indicative of focal dysmyelination (Tersar et al., 2007). As shown in Fig. 8D, the percentage of myelin infoldings and outfoldings in the day 14 SNI sciatic nerve was significantly reduced in Nox4^{-/-} mice as compared to WT mice, suggesting that injury-induced dysmyelination depends on Nox4. Altogether, the data show that ROS derived from Nox4 essentially contribute to the changes in myelination that occur in peripheral nerves as a response to nerve injury.

### References

Ahn DK, Lee SY, Han SR, Ju JS, Yang GY, Lee MK, Youn DH, Bae YC (2009) Intratrigeminal ganglionic injection of LPA causes neuropathic pain-like behavior and 25 demyelination in rats. Pain 146:114-120.
Anilkumar N, Weber R, Zhang M, Brewer A, Shah AM (2008) Nox4 and nox2 NADPH oxidases mediate distinct cellular redox signaling responses to agonist stimulation. Arterioscler Thromb Vasc Biol 28:1347-1354.
Basbaum AI, Bautista DM, Scherrer G, Julius D (2009) Cellular and molecular mechanisms of pain. Cell 139:267-284.
Bennett GJ, Xie YK (1988) A peripheral mononeuropathy in rat that produces disorders of pain sensation like those seen in man. Pain 33:87-107.
Berger JV, Deumens R, Goursaud S, Schafer S, Lavand'homme P, Joosten EA, Hermans E (2011) Enhanced neuroinflammation and pain hypersensitivity after peripheral nerve injury in rats expressing mutated superoxide dismutase 1. J Neuroinflamm 8: 33.
Braz JM, Nassar MA, Wood JN, Basbaum AI (2005) Parallel "pain" pathways arise from subpopulations of primary afferent nociceptor. Neuron 47:787-793.
Brown DI, Griendling KK (2009) Nox proteins in signal transduction. Free Radic Biol Med 47:1239-1253.
Casals-Diaz L, Vivo M, Navarro X (2009) Nociceptive responses and spinal plastic changes of afferent C-fibers in three neuropathic pain models induced by sciatic nerve injury in the rat. Exp Neurol 217:84-95.
Chen S, Velardez MO, Warot X, Yu ZX, Miller SJ, Cros D, Corfas G (2006) Neuregulin 1-erbB signaling is necessary for normal myelination and sensory function. The Journal of neuroscience : the official journal of the Society for Neuroscience 26:3079-3086.
Coderre TJ, Vaccarino AL, Melzack R (1990) Central nervous system plasticity in the tonic pain response to subcutaneous formalin injection. Brain Res 535:155-158.
Costigan M, Scholz J, Woolf CJ (2009) Neuropathic pain: a maladaptive response of the nervous system to damage. Annu Rev Neurosci 32:1-32.
Decosterd I, Woolf CJ (2000) Spared nerve injury: an animal model of persistent peripheral neuropathic pain. Pain 87:149-158.
Devor M (2006) Sodium channels and mechanisms of neuropathic pain. The journal of pain: official journal of the American Pain Society 7:S3-S12.
Diaz B, Shani G, Pass I, Anderson D, Quintavalle M, Courtneidge SA (2009) Tks5-dependent, nox-mediated generation of reactive oxygen species is necessary for invadopodia formation. Sci Signal 2:ra53.
Dray A (2008) Neuropathic pain: emerging treatments. Br J Anaesth 101:48-58. Dray A, Read SJ (2007) Arthritis and pain. Future targets to control osteoarthritis pain. Arthritis Res Ther 9:212.
Eberwine J, Yeh H, Miyashiro K, Cao Y, Nair S, Finnell R, Zettel M, Coleman P (1992) Analysis of gene expression in single live neurons. Proceedings of the National Academy of Sciences of the United States of America 89:3010-3014.
Ferreira J, Campos MM, Pesquero JB, Araujo RC, Bader M, Calixto JB (2001) Evidence for the participation of kinins in Freund's adjuvant-induced inflammatory and nociceptive responses in kinin B1 and B2 receptor knockout mice. Neuropharmacology 41:1006-1012.
Gao X, Kim HK, Chung JM, Chung K (2007) Reactive oxygen species (ROS) are involved in enhancement of NMDA-receptor phosphorylation in animal models of pain. Pain 131:262-271.
Gillespie CS, Sherman DL, Fleetwood-Walker SM, Cottrell DF, Tait S, Garry EM, Wallace VC, Ure J, Griffiths IR, Smith A, Brophy PJ (2000) Peripheral demyelination and neuropathic pain behavior in periaxin-deficient mice. Neuron 26:523-531.
Gorin Y, Ricono JM, Kim NH, Bhandari B, Choudhury GG, Abboud HE (2003) Nox4 mediates angiotensin II-induced activation of Akt/protein kinase B in mesangial cells. Am J Physiol Renal Physiol 285 :F219-229.
Hacimuftuoglu A, Handy CR, Goettl VM, Lin CG, Dane S, Stephens RL, Jr. (2006) Antioxidants attenuate multiple phases of formalin-induced nociceptive response in mice. Behav Brain Res 173:211-216.
Hunskaar S, Fasmer OB, Hole K (1985) Formalin test in mice, a useful technique for evaluating mild analgesics. J Neurosci Methods 14:69-76.
Ibi M, Matsuno K, Shiba D, Katsuyama M, Iwata K, Kakehi T, Nakagawa T, Sango K, Shirai Y, Yokoyama T, Kaneko S, Saito N, Yabe-Nishimura C (2008) Reactive oxygen species derived from NOX1/NADPH oxidase enhance inflammatory pain. J Neurosci 28:9486-9494.
Indra AK, Warot X, Brocard J, Bornert JM, Xiao JH, Chambon P, Metzger D (1999) Temporally-controlled site-specific mutagenesis in the basal layer of the epidermis: comparison of the recombinase activity of the tamoxifen-inducible Cre-ER(T) and Cre-ER(T2) recombinases. Nucleic Acids Res 27:4324-4327.
Inoue M, Rashid MH, Fujita R, Contos JJ, Chun J, Ueda H (2004) Initiation of neuropathic pain requires lysophosphatidic acid receptor signaling. Nature medicine 10:712-718.
Khattab MM (2006) TEMPOL, a membrane-permeable radical scavenger, attenuates peroxynitrite- and superoxide anion-enhanced carrageenan-induced paw edema and hyperalgesia: a key role for superoxide anion. Eur J Pharmacol 548:167-173.
Kim D, You B, Jo EK, Han SK, Simon MI, Lee SJ (2010) NADPH oxidase 2-derived reactive oxygen species in spinal cord microglia contribute to peripheral nerve injury-induced neuropathic pain. Proc Natl Acad Sci U S A 107:14851-14856.
Kim HK, Park SK, Zhou JL, Taglialatela G, Chung K, Coggeshall RE, Chung JM (2004) Reactive oxygen species (ROS) play an important role in a rat model of neuropathic pain. Pain 111:116-124.
Kim HY, Wang J, Lu Y, Chung JM, Chung K (2009) Superoxide signaling in pain is independent of nitric oxide signaling. Neuroreport 20:1424-1428.
Kobayashi H, Chattopadhyay S, Kato K, Dolkas J, Kikuchi S, Myers RR, Shubayev VI (2008) MMPs initiate Schwann cell-mediated MBP degradation and mechanical nociception after nerve damage. Molecular and cellular neurosciences 39:619-627.
Kuroda J, Nakagawa K, Yamasaki T, Nakamura K, Takeya R, Kuribayashi F, Imajoh-Ohmi S, Igarashi K, Shibata Y, Sueishi K, Sumimoto H (2005) The superoxide-producing NAD(P)H oxidase Nox4 in the nucleus of human vascular endothelial cells. Genes Cells 10:1139-1151.
Lambeth JD (2004) NOX enzymes and the biology of reactive oxygen. Nat Rev Immunol 4:181-189.
Lambeth JD, Kawahara T, Diebold B (2007) Regulation of Nox and Duox enzymatic activity and expression. Free Radic Biol Med 43:319-331.
Lee I, Kim HK, Kim JH, Chung K, Chung JM (2007) The role of reactive oxygen species in capsaicin-induced mechanical hyperalgesia and in the activities of dorsal horn neurons. Pain 133:9-17.
Lee KY, Chung K, Chung JM (2010) Involvement of reactive oxygen species in long-term potentiation in the spinal cord dorsal horn. J Neurophysiol 103:382-391.
Lee S, Gharavi NM, Honda H, Chang I, Kim B, Jen N, Li R, Zimman A, Berliner JA (2009) A role for NADPH oxidase 4 in the activation of vascular endothelial cells by oxidized phospholipids. Free Radic Biol Med 47:145-151.
Lyle AN, Deshpande NN, Taniyama Y, Seidel-Rogol B, Pounkova L, Du P, Papaharalambus C, Lassegue B, Griendling KK (2009) Poldip2, a novel regulator of Nox4 and cytoskeletal integrity in vascular smooth muscle cells. Circ Res 105:249-259.
Mahadev K, Motoshima H, Wu X, Ruddy JM, Arnold RS, Cheng G, Lambeth JD, Goldstein BJ (2004) The NAD(P)H oxidase homolog Nox4 modulates insulin-stimulated generation of H2O2 and plays an integral role in insulin signal transduction. Mol Cell Biol 24:1844-1854.
Majava V, Polverini E, Mazzini A, Nanekar R, Knoll W, Peters J, Natali F, Baumgartel P, Kursula I, Kursula P (2010) Structural and functional characterization of human peripheral nervous system myelin protein P2. PLoS One 5:e10300.
Marchesi C, Milani M, Morbin M, Cesani M, Lauria G, Scaioli V, Piccolo G, Fabrizi GM, Cavallaro T, Taroni F, Pareyson D (2010) Four novel cases of periaxin-related neuropathy and review of the literature. Neurology 75:1830-1838.
Meller ST, Gebhart GF (1997) Intraplantar zymosan as a reliable, quantifiable model of thermal and mechanical hyperalgesia in the rat. Eur J Pain 1:43-52.
Moalem-Taylor G, Allbutt HN, Iordanova MD, Tracey DJ (2007) Pain hypersensitivity in rats with experimental autoimmune neuritis, an animal model of human inflammatory demyelinating neuropathy. Brain Behav Immun 21:699-710.
Mogil JS, Wilson SG, Bon K, Lee SE, Chung K, Raber P, Pieper JO, Hain HS, Belknap JK, Hubert L, Elmer GI, Chung JM, Devor M (1999) Heritability of nociception I: responses of 11 inbred mouse strains on 12 measures of nociception. Pain 80:67-82.
Nagai J, Uchida H, Matsushita Y, Yano R, Ueda M, Niwa M, Aoki J, Chun J, Ueda H (2010) Autotaxin and lysophosphatidic acid1 receptor-mediated demyelination of dorsal root fibers by sciatic nerve injury and intrathecal lysophosphatidylcholine. Mol Pain 6:78.
Olechowski CJ, Truong JJ, Kerr BJ (2009) Neuropathic pain behaviours in a chronic relapsing model of experimental autoimmune encephalomyelitis (EAE). Pain 141:156-164.
Park HS, Jung HY, Park EY, Kim J, Lee WJ, Bae YS (2004) Cutting edge: direct interaction of TLR4 with NAD(P)H oxidase 4 isozyme is essential for lipopolysaccharide-induced production of reactive oxygen species and activation of NF-kappa B. J Immunol 173:3589-3593.
Schmidtko A, Gao W, Konig P, Heine S, Motterlini R, Ruth P, Schlossmann J, Koesling D, Niederberger E, Tegeder I, Friebe A, Geisslinger G (2008) cGMP produced by NOsensitive guanylyl cyclase essentially contributes to inflammatory and neuropathic pain by using targets different from cGMP-dependent protein kinase I. J Neurosci 28:8568-8576.
Schwartz ES, Lee I, Chung K, Chung JM (2008) Oxidative stress in the spinal cord is an important contributor in capsaicin-induced mechanical secondary hyperalgesia in mice. Pain 138:514-524.
Takac I, Schroder K, Zhang L, Lardy B, Anilkumar N, Lambeth JD, Shah AM, Morel F, Brandes RP (2011) The E-loop Is Involved in Hydrogen Peroxide Formation by the NADPH Oxidase Nox4. The Journal of biological chemistry 286:13304-13313.
Tanabe M, Nagatani Y, Saitoh K, Takasu K, Ono H (2009) Pharmacological assessments of nitric oxide synthase isoforms and downstream diversity of NO signaling in the maintenance of thermal and mechanical hypersensitivity after peripheral nerve injury in mice. Neuropharmacology 56:702-708.
Tersar K, Boentert M, Berger P, Bonneick S, Wessig C, Toyka KV, Young P, Suter U (2007) Mtmr13/Sbf2-deficient mice: an animal model for CMT4B2. Hum Mol Genet 16:2991-3001.
Ueda H (2008) Peripheral mechanisms of neuropathic pain - involvement of lysophosphatidic acid receptor-mediated demyelination. Mol Pain 4:11.
Vardeh D, Wang D, Costigan M, Lazarus M, Saper CB, Woolf CJ, Fitzgerald GA, Samad TA (2009) COX2 in CNS neural cells mediates mechanical inflammatory pain hypersensitivity in mice. J Clin Invest 119:287-294.
Wallace VC, Cottrell DF, Brophy PJ, Fleetwood-Walker SM (2003) Focal lysolecithin induced demyelination of peripheral afferents results in neuropathic pain behavior that is attenuated by cannabinoids. J Neurosci 23:3221-3233.
Wang C, Wu C, Popescu DC, Zhu J, Macklin WB, Miller RH, Wang Y (2011) Longitudinal near-infrared imaging of myelination. The Journal of neuroscience : the official journal of the Society for Neuroscience 31:2382-2390.
Zhang M, Brewer AC, Schroder K, Santos CX, Grieve DJ, Wang M, Anilkumar N, Yu B, Dong X, Walker SJ, Brandes RP, Shah AM (2010) NADPH oxidase-4 mediates protection against chronic load-induced stress in mouse hearts by enhancing angiogenesis. Proc Natl Acad Sci U S A 107:18121-18126.

## Claims

1. An *in vitro* method for identifying an inhibitor of NADPH-Oxidase 4 (Nox4) function and/or expression comprising the steps of:
(a) contacting a biological sample, comprising a nucleic acid sequence encoding for nox4, or the gene expression product of Nox4, with a candidate compound;
(b) assessing at least one of Nox4 activity or expression; and
(c) comparing the activity or expression in step (b) with the activity or expression of Nox4 in the absence of the candidate compound,
wherein a decrease between the measured activities or expression of Nox4 in step (b) compared to step (c) indicates that the candidate compound is an inhibitor of Nox4; and wherein a decrease between the measured activities or expression of Nox4 indicates that the candidate compound is for use in the prevention, treatment, of neuropathic pain.

2. The method according to claim, wherein the candidate compound is a protein, a peptide, a polypeptide, a polynucleotide, an oligonucleotide, or a chemical compound.

3. The method according to any one of claim 1 or 2, wherein the chemical compound is a pyrazolo pyridine derivative or a pyrazolo piperidine derivative.

4. The method according to any of claims 1 to 3, wherein assessing the activity of Nox4 comprises an enzyme activity assay, immunoassay, Western blotting, or assessing expression or activity of a gene regulated by Nox4, preferably the genes for matrix metalloproteinase-2 (MMP2), MAP kinase phosphatase-1 (MKP-1), p38 MAP kinase, or serum response factor (SRF).

5. The method according to any of claims 1 to 3, wherein assessing the expression of Nox4 comprises Northern blotting, microarray analysis, RT-PCR, or the assessment of reactive oxygen species (ROS) formation.

6. The method according to any one of claims 1 to 5 , wherein the neuropathic pain is associated with dysmyelination.

7. An inhibitor of NADPH-Oxidase 4 (Nox4) expression and/or function for use in the prevention and/or treatment of pain in a mammal, wherein the inhibitor of Nox4 is selected from antisense DNA- and/or RNA-oligonucleotides, antisense 2'-O-methyl oligoribonucleotides, antisense oligonucleotides containing phosphorothioate linkages, antisense oligonucleotides containing Locked Nucleic Acid LNA® bases, morpholino antisense oligos, small interfering RNA, miRNA, antagomirs, and mixtures thereof, and in particular an antagomir of Nox4.

8. The inhibitor for use of claim 7, for use in the prevention and/or treatment of neuropathic pain in a mammal.

9. A pharmaceutical composition comprising an inhibitor of Nox4 for use in the prevention and/or treatment of neuropathic pain, wherein the inhibitor of Nox4 is selected from antisense DNA- and/or RNA-oligonucleotides, antisense 2'-O-methyl oligoribonucleotides, antisense oligonucleotides containing phosphorothioate linkages, antisense oligonucleotides containing Locked Nucleic Acid LNA® bases, morpholino antisense oligos, small interfering RNA, miRNA, antagomirs, and mixtures thereof, and in particular an antagomir of Nox4.

10. The pharmaceutical composition for use according to claim 9, wherein the neuropathic pain is associated with dysmyelination.

11. The inhibitor for use according to any of claims 7 or 8, or a pharmaceutical composition for use according to any one of claims 9 or 10, wherein said inhibitor or pharmaceutical composition is administered orally, rectally, transmucosally, transdermally, intestinally, parenterally, intramuscularly, intrathecally, direct intraventricularly, intravenously, intraperitoneally, intranasally, intraocularly, or subcutaneously.

## Patentansprüche

1. Ein *in vitro* Verfahren zur Identifizierung eines Inhibitors der NADPH-Oxidase 4 (Nox4) Funktion und/oder Expression, umfassend die Schritte:
(a) In Kontakt bringen einer biologischen Probe, umfassend eine Nox4 kodierende Nukleinsäuresequenz, oder das Genexpressionsprodukt von Nox4, mit einer Kandidatenverbindung;
(b) Feststellen von zumindest der Nox4 Aktivität oder Expression; und
(c) Vergleichen der Aktivität oder Expression aus Schritt (b) mit der Aktivität oder Expression von Nox4 in Abwesenheit der Kandidatenverbindung,
wobei eine Verringerung der gemessenen Aktivitäten oder Expression von Nox4 in Schritt (b) im Vergleich zu Schritt (c) anzeigt, dass die Kandidatenverbindung ein Inhibitor von Nox4 ist; und wobei eine Verringerung der gemessenen Aktivitäten oder Expression von Nox4 anzeigt, dass die Kandidatenverbindung für die Prävention, Behandlung, von neuropathischem Schmerz geeignet ist.

2. Das Verfahren gemäß Anspruch 1 wobei die Kandidatenverbindung ein Protein, ein Peptid, ein Polypeptid, ein Polynukleotid, ein Oligonukleotid oder eine chemische Verbindung ist.

3. Das Verfahren gemäß Anspruch 1 oder 2, wobei die Kandidatenverbindung ein Pyrazolo-Pyridin Derivat oder ein Pyrazolo-Piperidin Derivat ist.

4. Das Verfahren gemäß einem der Ansprüche 1-3, wobei das Feststellen der Aktivität von Nox4 einen Enzym-Aktivitäts-Assay, einen Immuno-Assay, einen Western Blot oder das Feststellen der Expression oder Aktivität von durch Nox4 regulierten Genen umfasst, bevorzugt den Genen kodierend für Matrix-Metalloproteinas-2 (MMP2), MAP Kinase Phosphatase-1 (MKP1), p38 MAP Kinase oder "serum response factor" (SRF).

5. Das Verfahren gemäß einem der Ansprüche 1-3, wobei das Feststellen der Expression von Nox4 einen Northern Blot, eine Microarray Analyse, eine RT-PCR, oder die Feststellung der Bildung von reaktiven Sauerstoff Spezies (ROS), umfasst.

6. Das Verfahren gemäß einem der Ansprüche 1 bis 5, wobei der neuropathische Schmerz mit Dysmyelinisierung assoziiert ist.

7. Ein Inhibitor von Nox4 Expression und/oder Funktion zur Verwendung in der Prävention und/oder Behandlung von Schmerz in einem Säugetier, wobei der Inhibitor von Nox4 ausgewählt wird aus Antisense DNA- und/oder RNA-Oligonukleotiden, antisense 2'O-methyl Oligoribonukleotiden, antisense Oligonukleotiden, die Phosphorothiolat Verknüpfungen aufweisen, antisense Oligonukleotiden, die "Locked Nucleic Acid LNA® Basen aufweisen, Morpholino antisense Oligos, small interfering RNA, miRNA, Antagomire, und Mischungen davon, und im Besonderen ein Antagomir von Nox4.

8. Der Inhibitor zur Verwendung gemäß Anspruch 7 zur Verwendung in der Prävention und/oder der Behandlung von neuropathischem Schmerz in einem Säugetier.

9. Eine pharmazeutische Zusammensetzung umfassend ein Inhibitor von Nox4 zur Verwendung in der Prävention und/oder Behandlung von neuropathischem Schmerz, wobei der Inhibitor von Nox4 ausgewählt wird aus Antisense DNA- und/oder RNA-Oligonukleotiden, antisense 2'O-methyl Oligoribonukleotiden, antisense Oligonukleotiden, die Phosphorothiolat Verknüpfungen aufweisen, antisense Oligonukleotiden, die "Locked Nucleic Acid LNA® Basen aufweisen, Morpholino antisense Oligos, small interfering RNA, miRNA, Antagomire, und Mischungen davon, und im Besonderen ein Antagomir von Nox4.

10. Die pharmazeutische Zusammensetzung zur Verwendung gemäß Anspruch 9, wobei der neuropathische Schmerz mit Dysmyelinisierung assoziiert ist.

11. Der Inhibitor zur Verwendung gemäß einem der Ansprüche 7 oder 8, oder eine pharmazeutische Zusammensetzung zur Verwendung gemäß einem der Ansprüche 9 oder 10, wobei der genannte Inhibitor oder pharmazeutische Zusammensetzung oral, rektal, transmukosal, transdermal, intestinal, parenteral, intramuskulär, intrathekal, direkt intraventrikulär, intravenös, intraperitoneal, intranasal, intraokulär oder subkutan verabreicht wird.

## Revendications

1. Méthode d'identification *in vitro* d'un inhibiteur de la fonction et/ou de l'expression de la NADPH-oxydase 4 (Nox4) comprenant les étapes suivantes :
(a) la mise en contact d'un échantillon biologique, comprenant une séquence d'acide nucléique codant pour nox4, ou le produit d'expression génique de Nox4, avec un composé candidat ;
(b) l'évaluation d'au moins l'activité et/ou l'expression de Nox4 ; et
(c) la comparaison de l'activité ou de l'expression évaluée à l'étape (b) avec l'activité ou l'expression de Nox4 en l'absence du composé candidat,
dans laquelle une diminution entre les activités ou l'expression mesurées de Nox4 à l'étape (b) comparativement à l'étape (c) indique que le composé candidat est un inhibiteur de Nox4 ; et
dans laquelle une diminution entre les activités ou l'expression mesurées de Nox4 à l'étape (b) indique que le composé candidat peut être utilisé pour prévenir ou traiter la douleur neuropathique.

2. Méthode selon la revendication 1, dans laquelle le composé candidat est une protéine, un peptide, un polypeptide, un polynucléotide, un oligonucléotide, ou un composé chimique.

3. Méthode selon l'une quelconque des revendications 1 ou 2, dans laquelle le composé chimique est un dérivé de pyrazolopyridine ou un dérivé de pyrazolopipéridine.

4. Méthode selon l'une quelconque des revendications 1 à 3, dans laquelle l'évaluation de l'activité de Nox4 comprend un dosage de l'activité enzymatique, un dosage immunologique, un transfert Western, ou l'évaluation de l'expression ou de l'activité d'un gène régulé par Nox4, de préférence les gènes codant pour la métalloprotéinase matricielle 2 (MMP2), la MAP kinase phosphatase-1 (MKP-1), la MAP kinase p38, ou le facteur de réponse sérique (SRF).

5. Méthode selon l'une quelconque des revendications 1 à 3, dans laquelle l'évaluation de l'expression de Nox4 comprend un transfert Northern, une analyse sur micropuce, ou l'évaluation de la formation d'espèces réactives de l'oxygène (ROS).

6. Méthode selon l'une quelconque des revendications 1 à 5, dans laquelle la douleur neuropathique est associée à une démyélinisation.

7. Inhibiteur de l'expression et/ou de la fonction de la NADPH-oxydase 4 (Nox4) pouvant être utilisé pour prévenir et/ou traiter la douleur chez un mammifère, dans lequel l'inhibiteur de Nox4 est choisi parmi les oligonucléotides antisens de type ADN ou ARN, les oligoribonucléotides antisens 2'-O-méthyle, les oligonucléotides antisens contenant des liaisons phosphorothioate, les oligonucléotides antisens contenant des bases d'acides nucléiques verrouillés LNA®, des oligos antisens de type morpholino, un petit ARN d'interférence, un miARN, des antagomirs, et leurs mélanges, et en particulier un antagomir de Nox4.

8. Inhibiteur pour son utilisation selon la revendication 7, pouvant être utilisé pour prévenir et/ou traiter la douleur neuropathique chez un mammifère.

9. Composition pharmaceutique comprenant un inhibiteur de Nox4 pour son utilisation dans la prévention et/ou le traitement de la douleur neuropathique, dans laquelle l'inhibiteur de Nox4 est choisi parmi les oligonucléotides antisens de type ADN ou ARN, les oligoribonucléotides antisens 2'-O-méthyle, les oligonucléotides antisens contenant des liaisons phosphorothioate, les oligonucléotides antisens contenant des bases d'acides nucléiques verrouillés LNA®, des oligos antisens de type morpholino, un petit ARN d'interférence, un miARN, des antagomirs, et leurs mélanges, et en particulier un antagomir de Nox4.

10. Composition pharmaceutique pour son utilisation selon la revendication 9, dans laquelle la douleur neuropathique est associée à une démyélinisation.

11. Inhibiteur pour son utilisation selon la revendication 7 ou 8, ou composition pharmaceutique pour son utilisation selon l'une quelconque des revendications 9 ou 10, dans lequel ledit inhibiteur ou ladite composition pharmaceutique est administré(e) par voie orale, rectale, transmuqueuse, transdermique, intestinale, parentérale, intramusculaire, intracathétale, intraventriculaire directe, intraveineuse, intrapéritonéale, intranasale, intraoculaire, ou sous-cutanée.
